# EUROPEAN PATENT SPECIFICATION

(11) **EP 3 116 581 B1**
(45) Date of publication and mention of the grant of the patent: **19.06.2024**
(21) Application number: 15718391.4
(22) Date of filing: 13.03.2015
(51) Int. Cl.: A61K 45/06, A61K 31/436, A61K 31/337, A61K 31/727, A61P 7/02, A61P 9/00, A61P 35/00

(54) **IMPLANTABLE MEDICAL DEVICE HAVING COATINGS**
IMPLANTIERBARE MEDIZINISCHE VORRICHTUNG MIT ÜBERZÜGEN
DISPOSITIF MÉDICAL IMPLANTABLE AVEC DES REVETEMENTS

(30) Priority: 13.03.2014 US 201414210118
(43) Date of publication of application: 18.01.2017
(73) Proprietor: W. L. Gore & Associates, Inc., Newark, DE 19711 (US)
(72) Inventor: ANTONI, Per, S-194 61 Upplands Väsby (SE); LEONTEIN, Karin, S-194 61 Upplands Väsby (SE); VINCENT, Lars, S-194 61 Upplands Väsby (SE); CLEEK, Robert, L., Flagstaff, AZ 86004 (US); DRUMHELLER, Paul, D., Flagstaff, AZ 86001 (US); HOLLAND, Theresa, A., Flagstaff, AZ 86001 (US); JOHNSON, Todd, J., Flagstaff, AZ 86001 (US); PIETRZAK, Krzysztof, R., Flagstaff, AZ 86001 (US); STEINHAUS, Bruce, Flagstaff, AZ 86001 (US)
(74) Representative: Sagittarius IP
(86) International application number: PCT/US2015/020390
(87) International publication number: WO 2015/138862

(56) References cited:
- EP-A2- 2 198 814
- EP-A2- 2 228 082
- WO-A1-2008/106223
- WO-A2-03/074196
- US-A1- 2011 238 011
- BYUNG HA LEE ET AL: "Paclitaxel-coated expanded polytetrafluoroethylene haemodialysis grafts inhibit neointimal hyperplasia in porcine model of graft stenosis", NEPHROLOGY DIALYSIS TRANSPLANTATION, OXFORD UNIVERSITY PRESS, GB, vol. 21, no. 9, 1 September 2006 (2006-09-01), pages 2432-2438, XP002724871, ISSN: 0931-0509, DOI: 10.1093/NDT/GFL070 [retrieved on 2006-03-22]

## Description

### Field of the invention

The present invention relates to implantable medical devices with coatings comprising an immobilized heparin moiety and elutable paclitaxel and to methods for making such coated devices.

### Background of the invention

Implantable medical devices are frequently used to treat the anatomy of patients. Such devices can be permanently or semi-permanently implanted in the anatomy to provide treatment to the patient. Examples of such devices include stents, grafts, stent-grafts, filters, valves, occluders, markers, mapping devices, therapeutic agent delivery devices, prostheses, pumps, bandages, combinations thereof, and other endoluminal and implantable devices.

Implantable medical devices may be used to deliver a therapeutic agent in the vicinity of the implant, thereby providing localized as opposed to systemic delivery. Such devices can be described as drug-eluting devices. For example, in the case of localized vascular disease, systemic administration of a drug may not be desirable because the drug may have unwanted effects on parts of the body which are not to be treated, or because treatment of the diseased vasculature requires a high concentration of drug that may not be achievable by systemic administration. It is therefore often desirable to administer drugs in a localized manner to vascular tissues.

Several devices for localized drug delivery are known, including stents or stent-grafts which have been coated with an elutable drug.

A drug commonly used for the localized treatment of vascular disease, in particular for the prevention and treatment of restenosis, is paclitaxel. Paclitaxel can be coated onto the surface of a device using a variety of coating techniques. One technique involves combining the paclitaxel with an excipient, either in dry form using powder methods, or in solution, or in suspension using solvent methods. The paclitaxel-excipient combination is then applied to the surface of the device, either in the form of a powder or via the application of the solution or suspension followed by a drying step.

There are numerous factors that must be considered when creating a paclitaxel-excipient combination, and when coating the combination onto a medical device. In general, combining drugs and excipients and coating medical devices with drug-excipient combinations are complicated areas of technology. They involve the usual formulation challenges, such as those of oral or injectable pharmaceuticals, together with the added challenge of maintaining drug adherence to the medical device following compaction (if coated in its expanded state), as well as maintaining adherence until it reaches the target site and subsequently delivering the drug to the target tissues (i.e. deployment then elution) with the desired release and absorption kinetics. Chemical degradation of paclitaxel and the excipients upon storage must also be considered, and a further key requirement is that the paclitaxel, when formulated in the coating on an implantable medical device, must survive a sterilization process essentially intact, in particular an ethylene oxide sterilization process.

Thus, there is a need to develop paclitaxel-excipient combinations that are appropriate for the localized treatment of vascular disease. In particular, there is a need to develop coatings that have good adherence to the device during manufacturing processes and manipulation, but demonstrate optimum paclitaxel release characteristics when implanted in the target vascular tissue.

When a medical device is implanted in the body a number of different reactions are set into motion, some of them resulting in inflammation and some in the coagulation of the blood in contact with the device surface. In order to counteract these serious adverse effects, the well-known anti-coagulant compound heparin has for a long time been administered systemically to patients before the medical device is placed in their body, or when it is in contact with their body fluids, in order to provide an antithrombotic effect. Heparin-coated surfaces also tend to be biocompatible, thereby preventing/reducing inflammation.

Thrombin is one of several coagulation factors, all of which work together to result in the formation of thrombi at a surface in contact with the blood. Antithrombin (also known as antithrombin III) ("ATIII") is the most prominent coagulation inhibitor. It neutralizes the action of thrombin and other coagulation factors and thus restricts or limits blood coagulation. Heparin dramatically enhances the rate at which antithrombin inhibits coagulation factors. Heparin cofactor II ("HCII") is another coagulation factor which rapidly inhibits thrombin in the presence of heparin.

Systemic treatment with high doses of heparin is, however, often associated with serious side-effects of which bleeding is the predominant. Another rare, but serious complication of heparin therapy is the development of an allergic response called heparin induced thrombocytopenia that may lead to thrombosis (both venous and arterial). High-dose systemic heparin treatment e.g. during surgery also requires frequent monitoring of the activated clotting time (used to monitor and guide heparin therapy) and the corresponding dose adjustments as necessary.

Therefore, solutions have been sought where the need for a systemic heparinization of the patient would be unnecessary or can be limited. It was thought that this could be achieved through a surface modification of the medical devices using the anti-coagulative properties of heparin. Thus, a number of more or less successful technologies have been developed where a layer of heparin is attached to the surface of the medical device seeking thereby to render the surface non-thrombogenic. For devices where long term bioactivity is required, heparin should desirably be resistant to leaching and degradation.

Heparin is a polysaccharide carrying negatively charged sulfate and carboxylic acid groups on the saccharide units. Ionic binding of heparin to polycationic surfaces was thus attempted, but the surface modifications suffered from lack of stability resulting in lack of function, as the heparin leached from the surface. Thereafter, different surface modifications have been prepared wherein the heparin has been covalently bound to groups on the surface.

One of the most successful processes for rendering a medical device non-thrombogenic has been the covalent binding of a heparin fragment to a modified surface of the device. The general method and improvements thereof are described in various patent documents (see EP-B-0086186, EP-B-0086187, EP-B-0495820 and US 6,461,665).

These patents describe the preparation of a heparinised surface by reacting heparin modified to bear a terminal aldehyde groups with a surface on a medical device which has been modified to bear primary amino groups. An intermediate Schiff base is formed which is reduced *in situ* to form stable secondary amine linker, thereby covalently immobilizing the heparin.

Further methods for covalently attaching heparin to a surface while retaining its bioactivity are described in WO2010/029189, WO2011/110684 and WO2012/123384.

US2011238011 discloses a combination of balloon catheters and formulations containing active substances that adhere to the surface of the balloon membrane. EP2198814 discloses an implantable medical device comprising an intraluminal scaffold having a plurality of openings therein. WO2008106223 discloses an implantable medical device for delivering a therapeutic agent to the body tissue of a patient, and a method for making such a medical device. EP2228082 discloses a method for preparing a drug-eluting stent using chemical vapor deposition. WO03074196 discloses an apparatus for coating a medical device, the apparatus including a coating chamber, a vibrating structure within the coating chamber the vibrating structure capable of suspending a medical device positioned in the coating chamber, and a coating source, the coating source positioned to introduce coating into the coating chamber. Byung Ha Lee et al. 2006 21(9) 2432-2438 discloses that paclitaxel-coated expanded polytetrafluoroethylene haemodialysis grafts inhibit neointimal hyperplasia in a porcine model of graft stenosis.

### Summary of the invention

In one aspect, the present invention provides an implantable medical device with a surface having a coating comprising:
a first coating layer comprising an immobilized heparin moiety and
a second particulate coating layer comprising elutable paclitaxel and at least one organic additive,
wherein at least a portion of the second particulate coating layer is in contact with at least a portion of the first coating layer.

In another aspect, the present invention provides a process for preparing a coated implantable medical device comprising the steps of:
i) treating the medical device to provide a first coating layer comprising an immobilized heparin moiety; and further
ii) treating the medical device to provide a second particulate coating layer comprising elutable paclitaxel and at least one organic additive,
wherein at least a portion of the second particulate coating layer is in contact with at least a portion of the first coating layer.

### Brief description of the figures

- Figure 1:: shows platelet consumption for stent-grafts of the invention and comparators following the blood contact evaluation test (Example 20);
- Figure 2:: shows the absence of clot formation on stent-grafts of the invention following the blood contact evaluation test (Example 20);
- Figure 3:: shows a SEM image of the surface of a stent-graft of the invention before manipulation (Example 21);
- Figure 4:: shows a SEM image of the surface of a stent-graft of the invention after manipulation (Example 21).
- Figure 5:: shows dye-solvent casting of stent-grafts with and without a coating of immobilized heparin (first coating layer) (Example 23).
- Figure 6:: shows a stent with interconnecting fluoropolymer webs (as described in US2009/0182413).
- Figure 7:: shows a stent-graft consisting of a stent member and a graft member.
- Figure 8:: shows the paclitaxel content of a stent-graft of the invention and a comparator pre- and post-compaction and expansion (Example 5).
- Figure 9:: shows a coating arrangement wherein a tubular device is coated at both ends with the second particulate coating layer.
- Figure 10:: shows some possible first coating layer and second particulate coating layer arrangements in embodiments of the invention.

### Detailed description of the invention

### Implantable medical devices and materials

Implantable medical devices are devices which are implanted in the anatomy e.g. into the vasculature or other body lumen, space or cavity to provide a therapeutic effect. Implantable devices are left, in part or in whole, in the anatomy after the immediate surgical procedure to deliver them. Devices which are transiently inserted into a treatment region (i.e. inserted and then removed in the same surgical procedure), such as a medical balloon (for example, one inserted into the vasculature during a Percutaneous Transluminal Angioplasty (PTA)), are not considered to be implantable medical devices within the context of the present invention. Thus, the implantable medical device is not a medical balloon.

In one embodiment, the implantable medical device is a tubular medical device. Suitably the implantable medical device is an implantable endoluminal medical device, in particular an implantable endovascular medical device.

Examples of implantable medical devices include stents, grafts, stent-grafts, in-dwelling filters, valves, occluders, markers, mapping devices, therapeutic agent delivery devices, prostheses, pumps and bandages and other endoluminal and implantable devices.

Additional examples of implantable medical devices include indwelling monitoring chronic infusion lines, arterial lines, devices for continuous subarachnoid infusions, feeding tubes, CNS shunts (e.g., a ventriculopleural shunt, a ventriculo-atrial (VA) shunt, or a ventriculoperitoneal (VP) shunt), ventricular peritoneal shunts, ventricular atrial shunts, portosystemic shunts and shunts for ascites, devices for the filtering or removal of obstructions such as emboli and thrombi from blood vessels, dilation devices to restore patency to an occluded body passage, occlusion devices to selectively deliver a means to obstruct or fill a passage or space, and as a centering mechanism device for transluminal instruments like catheters.

Further examples of implantable medical devices of the present invention are catheters. Examples of catheters include, but are not limited to, central venous catheters, peripheral intravenous catheters, haemodialysis catheters, catheters such as coated catheters include implantable venous catheters, tunnelled venous catheters, coronary catheters useful for angiography, angioplasty, or ultrasound procedures in the heart or in peripheral veins and arteries, hepatic artery infusion catheters, CVC (central venous catheters), peripheral intravenous catheters, peripherally inserted central venous catheters (PIC lines), flow-directed balloon- tipped pulmonary artery catheters, total parenteral nutrition catheters, chronic dwelling catheters (e.g., chronic dwelling gastrointestinal catheters and chronic dwelling genitourinary catheters), peritoneal dialysis catheters, CPB catheters (cardiopulmonary bypass), urinary catheters and microcatheters (e.g. for intracranial application).

In one embodiment, the implantable medical device is a stent. In another embodiment, the implantable medical device is a stent-graft.

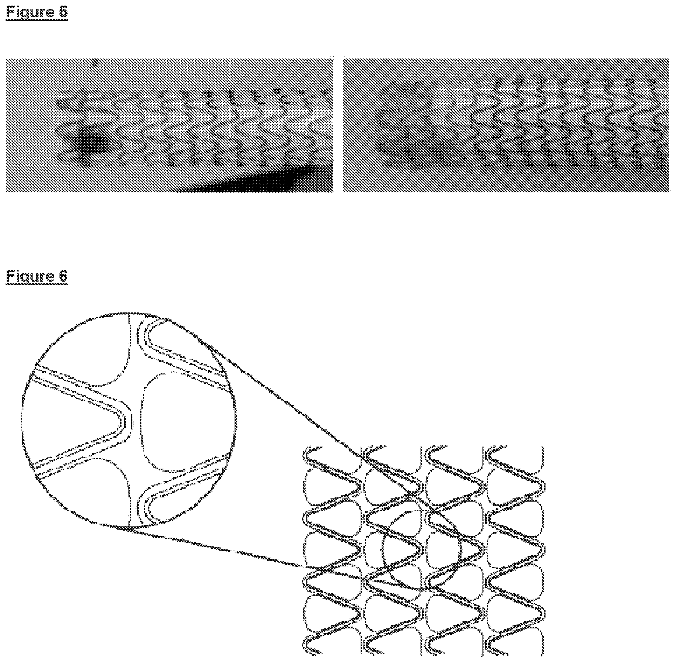

In one embodiment, the implantable medical device is a stent such as a bifurcated stent, balloon expandable stent or a self-expanding stent. Stents are configured as braids, wound wire forms, laser-cut forms, deposited materials, 3-D printed constructs, or combinations thereof, or take other structural forms, including those with length-adjustability, which provide support to a luminal wall or region. Stents are constructed of biocompatible materials including metals, metal alloys, such as stainless steel and nickel-titanium alloy (NiTi, or nitinol), polymers, ceramics, biodegradable materials (such as biodegradable polymers, ceramics, metals and metal alloys), or combinations thereof. Stents can be of substantially unitary form or comprise separate components, e.g., rings. Whether unitary or made up of components, stent structures can be joined together by struts, hinges, connectors, or materials which fully or partially line or cover the stent. In one embodiment, the stent structure is joined with fluoropolymers forming "webs" as described in US2009/0182413 (Gore Enterprise Holdings, Inc.) and illustrated in Figure 6.

In one embodiment, the medical device is a stent formed from a metal, a metal alloy, a polymer, a ceramic, a biodegradable material, or a combination thereof, in particular from a metal or a metal alloy. In one embodiment, the stent is formed from stainless steel. In another embodiment, the stent is formed from nitinol.

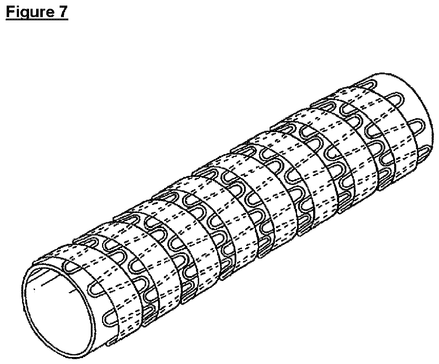

A stent-graft typically comprises a stent member and a graft member. Aspects described above with respect to stents apply equally to the stent members of stent grafts. Grafts are typically configured as tubular members, with closed walls or walls with openings. Graft materials include biocompatible materials such as fluoropolymers, including polytetrafluoroethylene (PTFE) and expanded polytetrafluoroethylene (ePTFE). Other suitable graft materials include polymers such as polyethylene terephthalate and ultra-high molecular weight polyethylene (UHMWPE). Graft materials can be made to possess different strengths, densities, dimensions, porosities and other functional characteristics and can take the form of films, extrusions, electrospun materials, coatings, depositions, or molded articles. Grafts may be used alone or graft materials can fully or partially line or cover a stent structure. In one embodiment, the stent-graft can take forms as described in US5,876,432 (Gore Enterprise Holdings, Inc.). A typical stent-graft construction is illustrated in Figure 7.

In another embodiment, the stent-graft is a vascular graft incorporating a stent into a portion of its length, as described in US2009/0076587 (Gore Enterprise Holdings, Inc.).

In one embodiment, the implantable medical device is a stent-graft, wherein the stent member comprises nitinol. In another embodiment, the implantable medical device is a stent-graft wherein the graft member is formed from a polymer, suitably a biocompatible polymer. Suitably the graft member is formed from a fluoropolymer such as expanded polytetrafluoroethylene (ePTFE). In one embodiment, the graft member is composed of ePTFE.

In one embodiment, the implantable medical device is a stent-graft comprising a stent member and a graft member, wherein the stent member comprises nitinol and the graft member comprises ePTFE.

Prior to coating with the first coating layer and second particulate coating layer, stents, stent-grafts and grafts can be overlain with various materials such as polymers and primer layers. In an embodiment, the stent or graft structure is modified to enhance the ability of the device to hold or release a therapeutic agent, in particular paclitaxel, applied to the device. For example, pits or blind holes can be formed in stent struts into which a therapeutic agent is loaded.

US2010/0152841 (Dave et al.) describes an expandable, non-removable medical device which can be implanted within a bodily lumen of a human or animal, with openings for delivery of a plurality of beneficial agents and a surface coating of antithrombotic agent. Also described are primer coatings for use between the antithrombotic agent coating and other therapeutic agent/polymer matrices.

In one embodiment, prior to coating the implantable medical device comprises a surface with, or is covered with, a porous material. In an embodiment, this porous material is a fluoropolymer such as polytetrafluoroethylene (PTFE), an expanded PTFE (ePTFE), fluorinated ethylene-propylene (FEP), perfluorocarbon copolymers, e.g. tetrafluoroethylene perfluoroalkylvinyl ether (TFE/PAVE) copolymers, copolymers of tetrafluoroethylene (TFE) and perfluoromethyl vinyl ether (PMVE), copolymers of TFE with functional monomers that comprise acetate, alcohol, amine, amide, sulfonate, functional groups and the like as described in U.S. Pat. No. 8,658,707 (W.L. Gore and Associates), as well as combinations thereof. The structure of expanded PTFE characterized by nodes interconnected by fibrils, is taught in U.S. Pat. Nos. 3,953,566 and 4,187,390 (W. L. Gore & Associates). In one embodiment, the porous material comprises ePTFE having a material structure with fibrils or fibrils and nodes. In another embodiment, the fibrils or fibrils and nodes change in size, dimension, or orientation as a dimension of the implantable medical device covering is changed. In another embodiment, the porous material is a knitted, woven or braided polyester.

In one embodiment, the coated implantable medical device has a covering disposed around at least a portion of a coating. Such a covering is typically described as a constraint or a sheath and is used to assist in the tracking and delivery of a medical device such as a stent or stent-graft. The constraint is considered to be a separate entity from the implantable medical device of the invention. In one embodiment, the constraint is disposed over the coated implantable medical device. The constraint can comprise any biocompatible material, including any possessing porosity or permeability. In one embodiment, the porosity or permeability varies as the material is deformed or otherwise altered in dimension.

The surface(s) or outward configuration of the constraint material may be modified with textures, protrusions, wires, blades, spikes, scorers, depressions, grooves, coatings, particles, and the like. These modifications may serve various purposes such as to modify tissues into which therapeutic agents will be (or have been) delivered, control placement of the system of the implantable medical device of the invention, and direct fluid transfer. Such textures may help in increased transfer of a therapeutic agent onto, more deeply and/or into deeper tissues. Such textures may be comprised of the covering material, or may be comprised of an added material.

The constraint may contain or be marked with radiopaque markers or be constructed to be radiopaque in its entirety. Such radiopaque indicators are used by clinicians to properly track and place an expandable medical device of the invention. As described in US8,753,386 (Shaw) optimal positioning of the stent-graft may be determined by various now known or as yet unknown techniques.

The implantable medical device, in particular a surface of the implantable medical device, can be composed of one or more materials as described hereinabove. The implantable medical device may comprise, consist, consist essentially of, or be formed of a metal or a synthetic or naturally occurring organic or inorganic polymer or a ceramic material, *inter alia.*

Thus, in one embodiment the implantable medical device, in particular a surface of the implantable medical device, is composed of a synthetic or naturally occurring organic or inorganic polymer or material, including but not limited to materials such as polyolefins, polyesters, polyurethanes, polyamides, polyether block amides, polyimides, polycarbonates, polyphenylene sulfides, polyphenylene oxides, polyethers, silicones, polycarbonates, polyhydroxyethylmethacrylate, polyvinyl pyrrolidone, polyvinyl alcohol, rubber, silicone rubber, polyhydroxyacids, polyallylamine, polyallylalcohol, polyacrylamide, and polyacrylic acid, styrenic polymers, polytetrafluoroethylene and copolymers thereof, expanded polytetrafluoroethylene and copolymers thereof, derivatives thereof and mixtures thereof. Some of these classes are available both as thermosets and as thermoplastic polymers. As used herein, the term "copolymer" shall be used to refer to any polymer formed from two or more monomers, e.g. 2, 3, 4, 5 and so on and so forth. Bioresorbables, such as poly(D,L-lactide) and polyglycolids and copolymers thereof are also useful. Non-woven, bioabsorbable web materials comprising a tri-block copolymer such as poly(glycolide-co-trimethylene carbonate) tri-block copolymer (PGA:TMC) are also useful (as described in US 7,659,219; Biran et al.). Useful polyamides include, but are not limited to, nylon 12, nylon 11, nylon 9, nylon 6/9 and nylon 6/6. Examples of some copolymers of such materials include the polyether-block-amides, available from Elf Atochem North America in Philadelphia, Pa. under the tradename of PEBAX^{®}. Another suitable copolymer is a polyetheresteramide. Suitable polyester copolymers, include, for example, polyethylene terephthalate and polybutylene terephthalate, polyester ethers and polyester elastomer copolymers such as those available from DuPont in Wilmington, Del. under the tradename of HYTREL.RTM. Block copolymer elastomers such as those copolymers having styrene end blocks, and midblocks formed from butadiene, isoprene, ethylene/butylene, ethylene/propene, and so forth may be employed herein. Other styrenic block copolymers include acrylonitrile-styrene and acrylonitrile-butadiene-styrene block copolymers. Also, block copolymers wherein the particular block copolymer thermoplastic elastomers in which the block copolymer is made up of hard segments of a polyester or polyamide and soft segments of polyether may also be employed herein. Other useful materials are polystyrenes, poly(methyl)methacrylates, polyacrylonitriles, poly(vinylacetates), poly(vinyl alcohols), chlorine-containing polymers such as poly(vinyl) chloride, polyoxymethylenes, polycarbonates, polyamides, polyimides, polyurethanes, phenolics, amino-epoxy resins, polyesters, silicones, cellulose-based plastics, and rubber-like plastics. Combinations of these materials can be employed with and without cross-linking. Polymeric materials may optionally be blended with fillers and/or colorants, such as a gold, barium, or tantalum filler to render the polymeric material radiopaque. Polymeric materials may optionally be modified at their surface while retaining bulk properties using methods known in the art, such as acid or base etching, hydrolysis, aminolysis, plasma modification, plasma grafting, corona discharge modification, chemical vapour deposition, ion implantation, ion sputtering, ozonation, photomodification, electron beam modification, gamma beam modification, and the like. In one embodiment, a surface of the medical device is composed of nylon.

The implantable medical device, in particular a surface of the implantable medical device, may be composed of fluorinated polymers such as fluoropolymers, e.g. expanded polytetrafluoroethylene (ePTFE), polytetrafluoroethylene (PTFE), fluorinated ethylene-propylene (FEP), perfluorocarbon copolymers, e.g. tetrafluoroethylene perfluoroalkylvinyl ether (TFE/PAVE) copolymers, copolymers of tetrafluoroethylene (TFE) and perfluoromethyl vinyl ether (PMVE), copolymers of TFE with functional monomers that comprise acetate, alcohol, amine, amide, sulfonate, functional groups and the like as described in U.S. Pat. No. 8,658,707 (W.L. Gore and Associates), as well as combinations thereof. Also contemplated are combinations of the above with and without crosslinking between the polymer chains, expanded polyethylene, polyvinylchloride, polyurethane, silicone, polyethylene, polypropylene, polyurethane, polyglycolic acid, polyesters, polyamides, elastomers and their mixtures, blends and copolymers or derivatives thereof. ePTFE has a porous microstructure which is particularly useful in the medical device of the invention. In one embodiment, the implantable medical device comprises ePTFE. Suitably a surface of the implantable medical device is composed of ePTFE. The exact porosity of a device composed of ePTFE will depend on the nature of the ePTFE components and the manner in which the device is processed. The porosity of a device composed of ePTFE can be evaluated using various methods and parameters, as described in US2013/0231733 (W.L. Gore & Associates, Inc.).

The implantable medical device, in particular a surface of the implantable medical device, may also be composed of metals, including, but are not limited to, biocompatible metals, titanium, stainless steel, high nitrogen stainless steel, gold, silver, rhodium, zinc, platinum, rubidium, copper and magnesium, and combinations thereof. Suitable alloys include cobalt alloys including cobalt-chromium alloys such as L-605, MP35N, Elgiloy, titanium alloys including nickel-titanium alloys (such as Nitinol), tantalum, and niobium alloys, such as Nb-1% Zr, and others. In one embodiment, the medical device is a stent and is composed of biocompatible metal selected from stainless steel, tantalum, titanium alloys and cobalt alloys. The implantable medical device, in particular a surface of the implantable medical device, may also be composed of a ceramic substrate including, but are not limited to, silicone oxides, aluminum oxides, alumina, silica, hydroxyapatites, glasses, calcium oxides, polysilanols, and phosphorous oxide.

### Coating layer

The coating on the implantable medical device of the present invention comprises a first coating layer comprising an immobilized heparin moiety and a second particulate coating layer comprising elutable paclitaxel and at least one organic additive.

### First coating layer

The first coating layer comprises an immobilized heparin moiety. The term "heparin moiety" refers to a heparin molecule, a fragment of the heparin molecule, or a derivative or analogue of heparin. Heparin derivatives can be any functional or structural variation of heparin. Representative variations include alkali metal or alkaline earth metal salts of heparin, such as sodium heparin (e.g. Hepsal or Pularin), potassium heparin (e.g. Clarin), lithium heparin, calcium heparin (e.g. Calciparine), magnesium heparin (e.g. Cutheparine), and low molecular weight heparin (prepared by e.g. oxidative depolymerisationdepolymerisation, enzymatic degradation or deaminative cleavage, e.g. ardeparin sodium, tinzaparin or dalteparin). Other examples include heparan sulfate, heparinoids, heparin-based compounds and heparin having a hydrophobic counter-ion. Other desirable anti-coagulant entities include synthetic heparin compositions referred to as "fondaparinux" compositions (e.g. Arixtra from GlaxoSmithKline) involving antithrombin-mediated inhibition of factor Xa. Additional derivatives of heparin include heparins and heparin moieties modified by means of e.g. mild nitrous acid degradation (US4,613,665) or periodate oxidation (US 6,653,457) and other modification reactions known in the art where the bioactivity of the heparin moiety is preserved. Heparin moieties also include such moieties bound to a linker or spacer as described below.

In one embodiment, the heparin moiety is full length heparin.

De-sulphated heparin, and heparin functionalized via e.g. the carboxylic acid group of the uronic acid moiety, are less suitable than other forms of heparin because of their generally reduced anti-coagulant properties relative to other forms of heparin. Thus, in one embodiment the heparin moiety is not de-sulphated heparin. Mono-functionalization or low functionalization degrees of carboxylic acid groups can be acceptable as long as heparin bioactivity is preserved.

US 6,461,665 (Scholander) discloses improving the antithrombogenic activity of surface-immobilized heparin by treating the heparin prior to immobilization. The improvement is achieved by treating the heparin at elevated temperature or at elevated pH, or by contacting the heparin with nucleophilic catalysts such as amines, alcohols, thiols or immobilized amino, hydroxyl or thiol groups.

The heparin moiety is "immobilized" in the sense that substantially all of it remains attached to the device (as part of the first coating layer) for the lifetime of the device. The heparin moiety does not substantially elute or leach from the first coating layer. As discussed below, the heparin moiety can be immobilized by various methods. Preferably the heparin moiety is covalently immobilized.

The heparin moiety is immobilized on a surface of the implantable medical device, preferably via immobilization on a polymer. Thus, in one embodiment, the first coating layer comprises a polymer. In another embodiment, the first coating layer comprises a polymer wherein the heparin moiety is attached to the polymer. In this embodiment, the polymer can be applied to the medical device, followed by immobilization of the heparin moiety to the polymer. Alternatively, the heparin moiety can firstly be immobilized to the polymer, and then the immobilized polymer-heparin moiety conjugate can be applied to the surface of the implantable medical device.

Thus, in one embodiment, the first coating layer is formed by a process comprising the steps of:
a) treating the medical device to provide a polymer coating layer; and then
b) reacting said polymer layer with a heparin moiety to immobilize the heparin moiety to the polymer coating layer.

In another embodiment, the first coating layer is formed by a process comprising the steps of:
a) reacting a polymer with a heparin moiety, thereby to form a polymer-heparin moiety conjugate wherein the heparin moiety is immobilized to the polymer;
b) treating the medical device with the polymer-heparin moiety conjugate of step a).

The heparin moiety may be immobilized to the polymer by various methods. In one embodiment, the heparin moiety is covalently attached to a polymer. Suitably the heparin moiety is covalently end-point attached to the polymer, preferably connected through the reducing end (position C1 of the reducing terminal) of the heparin moiety.

A representative end-point attachment process is described in EP-B-0086186 (Larm) which discloses a process for the covalent binding of oligomeric or polymeric organic substances to substrates of different types containing primary amino groups. The substance to be coupled, which may be heparin, is subjected to degradation by diazotation to form a substance fragment having a free terminal aldehyde group. The substance fragment is then reacted through its aldehyde group with the amino group of the substrate to form a Schiff's base, which is then converted (via reduction) to a secondary amine. The advantage of end point attachment of heparin, especially reducing end point attachment (as described above in EP-B-0086186), is that the biological activity of the heparin moiety is maximized due to enhanced availability of the antithrombin interaction sites as compared with attachment elsewhere in heparin moiety.

The nature of the polymer upon which the heparin moiety is immobilized can impact the resulting biological activity of the heparin moiety. EP-B-0086187 (Gölander et al.) describes the immobilization of anionic compounds, including heparin, to a substrate carrying a complex of a polymeric cationic surfactant containing primary amine groups and a dialdehyde as a cross-linking agent. The addition of the dialdehyde provides improved surfactant characteristics and can lead to surprisingly strong electrostatic binding with the anionic compounds. Examples wherein heparin is firmly bonded to the complex via covalent or ionic bonding are described. EP-B-0495820 (Larm et al.), building on the teaching of Gölander et al., describes that when crotonaldehyde is used as a cross-linking agent in the complex rather than glutaraldehyde, heparin which has been covalently attached to the surface (as described in EP-B-0086186 above) has improved activity.

Thus, in one embodiment, the first coating layer comprises a cationic polymer, typically a polyamine. In another embodiment, the cationic polymer is cross-linked.

The heparin moiety may be covalently bonded to the polymer via a linker other than the secondary amine described in EP-B-0086186 above. WO2010/029189 (Carmeda AB) describes the covalent attachment of an anticoagulant such as heparin to surface via a 1,2,3-trizole linkage. The document describes the azide- or alkyne-functionalization of a polyimine; the preparation of alkyne- and azide-functionalized heparin (both native and nitrous acid degraded heparin); and reactions to link the derivatised heparin to the derivatised polymer via a 1,2,3-triazole linker. WO2011/110684 (Carmeda AB et al.) describes the covalent attachment of an anticoagulant entity such as heparin to a polymer surface via a linker comprising a thioether.

WO2012/123384 (Gore Enterprise Holdings, Inc. et al.) discloses a device with a coating comprising a plurality of hyperbranched polymer molecules bearing anticoagulant entities, in particular heparin.

A polymer upon which the heparin moiety is immobilized, in particular a cationic polymer upon which the heparin moiety is covalently attached, may itself be disposed upon one or more coating bilayers of cationic polymer and anionic polymer.

Thus, in one embodiment the first coating layer comprises one or more coating bilayers of cationic polymer and anionic polymer, the innermost layer being a layer of cationic polymer and the outermost layer being an outer coating layer of cationic polymer to which the heparin moiety is covalently attached.

The first coating layer may comprise one or more coating bilayers, e.g. 2 or more, or 3 or 4 or 5 e.g. up to 20 coating bilayers such that desirably a portion of the surface (desired to be non-thrombogenic) or the whole of the surface of the object is covered (Multilayer Thin Films ISBN: 978-3-527-30440-0). The optimum number of bilayers will depend on the type of material from which the object is made, and the contemplated use of the surface. The surface may, if desired, be made up layer by layer. The number and nature of the bilayers needed to provide a full coverage of the surface can be easily determined by those skilled in the art. The coating bilayer(s) may be formed by adsorbing on the surface of the solid object high average molecular weight cationic polymer, e.g. a polyamine (e.g. polyethyleneimine e.g. as used in the examples of EP0495820B1 (Norsk Hydro A/S) and if needed cross-linking the polyamine with, e.g. an aldehyde crosslinker such as crotonaldehyde and/or glutaraldehyde (see EP-B-0086187 and EP-B-0495820 above), followed by the application of a solution of an anionic polymer, e.g. an anionic polysaccharide, e.g. dextran sulfate, to obtain at least one adsorbed layer of the polysaccharide. Hence the first coating layer may comprise a layer of high average molecular weight polyamine and a layer of anionic polysaccharide. More generally, the first coating layer may comprise one or more coating bilayers of cationic polymer (e.g. polyamine) and anionic polymer (e.g. anionic polysaccharide), the innermost layer being a layer of cationic polymer and the outer layer being a layer of cationic polymer to which the heparin moiety is immobilized. This coating procedure is performed essentially as described in EP-B-0495820 (see above). Thus, theoretically it is only the outer coating layer (within the first coating layer) which comprises the immobilized heparin moiety. Typically, the outer coating layer (of the first coating layer) to which the heparin moiety is immobilized is not cross-linked. The procedure of EP-B-0495820 (see above) may however be modified so that the outer layer (of the first coating layer) is the anionic polysaccharide which is then reacted, as described below, with a polyamine to which is immobilized the heparin moiety entity.

Various methods for preparing the first coating layer are described in Example 1.

In an alternative embodiment wherein the first coating layer comprises a polymer, the polymer is an anionic polymer, such as albumin. US4,526,714 (Cordis Europa N.V.) describes the preparation of heparin-albumin conjugates.

In one embodiment, the first coating layer consists of a polymer and a heparin moiety, suitably wherein the heparin moiety is covalently attached to the polymer.

Typically, the first coating layer will have an average total thickness of about 5 nm to about 1000 nm, such as about 10 nm to about 500 nm. Coating thickness can be measured using a suitable coating thickness analyser or gauge, or by using X-ray photoelectron spectroscopy with depth profiling (see Evaluation Methods).

### Second particulate coating layer

The second particulate coating layer comprises elutable paclitaxel and at least one organic additive.

Paclitaxel is sold commercially in formulations for the treatment of various cancers and for the prevention and treatment of restenosis. Paclitaxel is known to exist in several different physical forms, including amorphous, glassy and crystalline forms, wherein the crystalline forms can be further differentiated into a number of different polymorphs. Furthermore, crystalline paclitaxel can exist as an anhydrate or in hydrated form, e.g. as paclitaxel dihydrate. In one embodiment, the paclitaxel is anhydrous paclitaxel. In another embodiment, the paclitaxel is paclitaxel hydrate. In another embodiment, the paclitaxel is paclitaxel dihydrate. In an alternative embodiment, both anhydrous and hydrated forms of paclitaxel may be used. It should be noted that when paclitaxel is dissolved in an aqueous or partially aqueous solvent, practically speaking it is not of importance whether the starting paclitaxel is in the form of the hydrate or anhydrate, since the difference in water content between the two forms will be minimal, compared with the overall water content of the solution in which it is dissolved.

The accepted melting point of crystalline paclitaxel is circa 220 °C, depending on the heating conditions and polymorph form (Liggins et al. "Solid-state characterization of paclitaxel", J. Pharm. Sci. 1997, Vol. 86, pages 1458-1463). It is known that the particular form of paclitaxel can affect the physical properties of the drug when in solid form. In particular, the adherence of paclitaxel to a surface may be influenced by its physical form, as can its rate of dissolution (i.e. elution) from a surface to the surroundings. Thus, formulating paclitaxel for solid delivery can be challenging at the first instance, and the effect of formulating paclitaxel in solid form with an excipient cannot easily be predicted.

The second particulate coating layer also comprises at least one organic additive. The organic additive is also sometimes referred to herein as the "excipient". The organic additive is preferably non-polymeric. In one embodiment, the second particulate coating layer is polymer-free. The term "non-polymeric" will be clear to a person of skill in the art as meaning a substance which does not contain multiple repeating monomer units. Typically, a polymer will consist of at least 5 repeating monomer units, for example at least 6, at least 7, at least 8 or at least 9 repeating monomer units. References to polymers are intended to include copolymers. Examples of polymeric substances include proteins which are thus not suitable as organic additives for use in the invention. Poly(lactic-co-glycolic) acid (PLGA), polyvinylpyrrolidone (PVP) and polyethylene glycol (PEG) and poloxamers are examples of polymers which are not suitable as an organic additive for use in the invention. In particular poly(lactic-co-glycolic) acid (PLGA), polyvinylpyrrolidone (PVP) or polyethylene glycol (PEG) are not suitable for use as an organic additive. A further example of a material which is polymeric and therefore not suitable as an organic additive in the second particulate coating layer is shellac.

In one embodiment, the organic additive is not a plasticizer. In another embodiment, the second particulate coating layer is plasticizer-free i.e. does not contain a plasticizer. Plasticizers (also known as dispersants) are defined herein as compounds that increase the plasticity or fluidity of a material, usually a polymer. Plasticizers can be in monomeric, oligomeric or polymeric form. Examples of plasticizers include acetic acid, formic acid, 1-butanol, 2-butanol, ethanol, 2-methyl-1-butanol, 2-methyl-1-propanol, 1-pentanol, 1-propanol, 2-propanol, ethyl acetate, ethyl formate, isopropyl acetate, methyl acetate, propyl acetate, anisole, tert-butylmethyl ether, ethyl ether, cumene, heptane, pentane, acetone, methylethyl ketone, methylisobutyl ketone, dimethyl sulfoxide, glycerin, polyethylene glycols, polyethylene glycol monomethyl ether, sorbitol, sorbitan, citrate esters including acetyl tributyl citrate, acetyl triethyl citrate, tributyl citrate, triethyl citrate and the like, castor oil, diacetylated monoglycerides, dibutyl sebacate, diethyl phthalate, triacetin, fractionated coconut oil, and acetylated monoglycerides.

The organic additive is preferably hydrolytically stable i.e. resistant to chemical reaction/decomposition in the presence of water. A compound which is not hydrolytically stable will undergo an irreversible chemical transformation in an aqueous solution e.g. ester or amide or anhydride hydrolysis. Conversely, a compound which is hydrolytically stable will not undergo an irreversible transformation in an aqueous solution. A compound may undergo reversible proton exchange, or reversible hydrate formation and still be considered as being hydrolytically stable. When a compound is exposed to an aqueous solution and a chemical transformation results, and if the resulting compound (degradant) cannot be converted back to the original compound by simple pH modification, then the original compound is not hydrolytically stable.

In one embodiment, a compound is hydrolytically stable if, when exposed to buffered saline at pH 7.4, for 1 to 24 h (for example 5 h, 10 h, 15 h or 24 h), it does not show chemical reaction or degradation when analyzed with ultra-performance liquid chromatography (UPLC; see Evaluation Methods) or high-performance liquid chromatography. In one embodiment, a compound is considered to be hydrolytically stable if, following the treatment above, at least 80%, for example at least 90% or 95% of the compound is recovered in un-degraded form.

Whether a particular compound is hydrolytically stable or not can depend on the pH. In one embodiment, the organic additive is hydrolytically stable at physiological pH. In one embodiment, physiological pH is pH 7.4.

Certain chemical functional groups such as esters, in particular succinimidyl esters, sulfosuccinimidyl esters, acyl halides, acetals, hemiacetals, and anhydrides are known to be prone to hydrolysis therefore compounds containing such functionality might, at the first instance, appear not be suitable as an organic additive. However, the hydrolytic stability of such functional groups can be enhanced by the remaining functionality of the compound e.g. by steric or electronic effects of neighbouring functional groups. Thus, although the presence of functional groups known to be prone to hydrolysis may, on a first assessment, indicate that a compound is unsuitable for the purposes of being the organic additive, the compound as a whole should be assessed. The following substances at least are not suitable as organic additives for use in the present invention because they are not hydrolytically stable: gluconolactone, maleic anhydride, diglycolic anhydride and acetic anhydride.

Early experiments indicated that vanillin was unsuitable for use as the organic additive as it easily degraded in solution. Thus, vanillin is not suitable as an organic additive for the present invention. In one embodiment, the second particulate coating layer does not contain vanillin. In one embodiment, the organic additive does not contain phenolic aldehyde functionality. In another embodiment, the second particulate coating layer does not contain compounds containing phenolic aldehyde functionality.

The use of Hansen solubility parameters can assist in the understanding or rationalization of the behaviour of a composition comprising two or more components (Mohammed et al. International Journal of Pharmaceutics 2011, Vol. 407 pp 63-71 and Albers et al. Journal of Pharmaceutical Sciences 2011, Vol. 100 pp 667-680). In one embodiment, the organic additive is a substance having a value for the dispersion component of the Hansen solubility parameter determined at 25 °C substantially the same as that of paclitaxel. In one embodiment, "substantially the same" means within ±3.0 MPa^{0.5} of the value for the dispersion component of the Hansen solubility parameter for paclitaxel (determined at 25 °C). Suitably the dispersion component of the Hansen solubility parameter determined at 25 °C of the organic additive is between 16 and 21 MPa^{0.5}.

The organic additive will typically have a low molecular weight. For example, the organic additive will have a molecular weight of less than 1200 Da, less than 990 Da, less than 750 Da, less than 500 Da, less than 400 Da or less than 300 Da. In one embodiment, the organic additive has a molecular weight of between about 50 Da and about 400 Da, for example between about 80 Da and about 350 Da. The organic additive is not a protein. In one embodiment, the second particulate coating layer is free of protein. In a further embodiment, the organic additive is not a therapeutic agent. In an embodiment, the organic additive is not aspirin.

The organic additive will typically have a melting point of greater than 80 °C when in pure form, for example greater than 90 °C, greater than 100 °C, greater than 110 °C or greater than 120 °C. Compounds with a melting point lower than 80 °C when in pure form generally have weak intermolecular interactions, potentially leading to the compound being physically unstable. Compounds that are capable of forming coordinated solvates, such as a hydrate, with the paclitaxel and/or the organic additive typically have physical stability.

In one embodiment the or each (at least one) organic additive is independently selected from the list consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, adipic acid, glutaric acid, theophylline, and saccharin sodium. The formation of a second particulate coating layer using caffeine or succinic acid as organic additive is described in Examples 2, 3, 3a, 3b and 3c. Suitably the or each (at least one) organic additive is independently selected from the list consisting of p-aminobenzoic acid, methyl paraben, caffeine, calcium salicylate and succinic acid. In one embodiment the organic additive is succinic acid. In another embodiment, the organic additive is caffeine.

In an embodiment the organic additive is not sodium salicylate. In an embodiment the organic additive is not calcium salicylate. In an embodiment the organic additive is not magnesium salicylate. In an embodiment the second particulate coating layer does not contain sodium salicylate. In an embodiment the second particulate coating layer does not contain calcium salicylate. In an embodiment the second particulate coating layer does not contain magnesium salicylate.

In an embodiment the organic additive is not a substance containing magnesium ions i.e. a magnesium salt. In an embodiment the second particulate coating layer does not contain magnesium ions i.e. a magnesium salt.

In an embodiment the organic additive is not ascorbic acid or a salt thereof e.g. L-ascorbic acid or a salt thereof. In one embodiment the second particulate coating layer does not contain L-ascorbic acid or a salt thereof.

The paclitaxel when formulated in the second particulate coating layer (and indeed the entire coating layer) should suitably be able to withstand a sterilization process essentially intact. Thus, in one embodiment, the paclitaxel, when formulated in the second particulate coating layer with the or each organic additive, is stable to sterilization.

Compounds with certain functional groups such as primary amides (-C(O)NH₂) and primary alkyl amines (alkyl-NH₂) have been observed to be incompatible with paclitaxel, when formulated together as a solid coating and subjected to ethylene oxide sterilization. An example of such a compound is niacinamide, which when formulated with paclitaxel and coated onto a balloon, resulted in nearly complete paclitaxel degradation when the balloon was ethylene oxide sterilised. Thus, compounds containing such functionality might cause paclitaxel to degrade under ethylene oxide sterilization, therefore might not be suitable as an organic additive in the implantable medical device of the invention. However, the interaction of such compounds with paclitaxel may be altered by the remaining functionality of the molecule, for example, the reactivity of primary amide or primary alkyl amine groups adjacent to aromatic functionality can be tempered to the extent that such compounds will not cause degradation of paclitaxel under ethylene oxide sterilization conditions. The following substances at least are not suitable as organic additives for use in the present invention because they cause degradation of the paclitaxel under the conditions of ethylene oxide sterilization: niacinamide and sodium salicylate.

Thus, the organic additive is not niacinamide (also known as nicotinamide) or sodium salicylate. In one embodiment, the second particulate coating layer does not contain niacinamide or sodium salicyclate.

As discussed in further detail below, implantable medical devices of the invention can be prepared by dipping the medical device (suitably pre-coated with the first coating layer comprising an immobilized heparin moiety) into a solution containing paclitaxel and the at least one organic additive. Using this method it is difficult to achieve a suitable coating unless both components are soluble in the solution. The present inventors found it was not possible to form coatings where the organic additive has poor solubility in the solvent system. For example, thiamine-HCl has poor solubility in acetone, ethanol and aqueous mixtures thereof and attempts to formulate a paclitaxel-thiamine-HCl coating on a balloon were unsuccessful. Thus, in one embodiment the organic additive is not thiamine-HCl. In another embodiment, the second particulate coating layer does not contain thiamine-HCl.

Suitably the organic additive is not dexpanthenol. Suitably the organic additive is not ricinoleic acid. Suitably the organic additive is not resorcin. Suitably the organic additive is not isomalt. Suitably the second particulate coating layer does not contain dexpanthenol, recinoleic acid, resorcin or isomalt.

In one embodiment, the organic additive is not a surfactant. In one embodiment, the second particulate coating layer is surfactant-free i.e. does not contain a surfactant. Surfactants are defined herein as compounds that are amphiphilic and contain both hydrophobic and hydrophilic groups and include ionic, non-ionic, zwitterionic, aliphatic and aromatic surfactants. Surfactants can be in monomeric, oligomeric or polymeric form. Examples of surfactants include, but are not limited to, polysorbate (Tween^{®} 20, Tween^{®} 40, Tween^{®} 60), PEG-fatty esters, PEG mega-3 fatty esters, PEG ethers (such as Triton X-100/octoxynol-9) and alcohols (such as tyloxapol), glycerol fatty esters, sorbitan fatty esters, PEG, glyceryl fatty esters, PEG sorbitan fatty esters, PEG sugar esters, poloxamers (which may be sold under the trade names of Synperonics^{®}, Pluronics^{®} and Kolliphor^{®}), ascorbyl palmitate and p- isononylphenoxypolyglycidol (Olin 10-G^{®} or Surfactant 10-G^{®}).

In one embodiment, the second particulate coating layer is free of cyclodextrin.

In one embodiment, the second particulate coating layer is free of inorganic components (e.g. salts having both inorganic cations and inorganic anions). Suitably the second particulate coating layer is bioabsorbable or is biostable.

In one embodiment, the second particulate coating layer consists of paclitaxel and at least one organic additive. In this embodiment, the second particulate coating layer does not comprise components other than paclitaxel and the or each organic additive(s) as described herein.

In one embodiment, the second particulate coating layer comprises one organic additive. In one embodiment, the second particulate coating layer consists of paclitaxel and one organic additive as described herein. In this embodiment, the second particulate coating layer is a binary composition (as described in Examples 2, 3, 3a, 3b and 3c). In one embodiment, the organic additive is succinic acid. In another embodiment, the organic additive is caffeine.

In one embodiment, the second particulate coating layer comprises two organic additives. In one embodiment, the second particulate coating layer consists of paclitaxel and two organic additives as described herein. In this embodiment, the second particulate coating layer is a ternary composition. In one embodiment, the two organic additives are caffeine and succinic acid. In one embodiment, the second particulate coating layer comprises three or more organic additives.

In another embodiment, the second particulate coating layer is not a ternary composition i.e. the second particulate coating layer consists of more or fewer than three components. In another embodiment, the second particulate coating layer is not a quaternary composition i.e. the second particulate coating layer consists of more or fewer than four components.

The particulate nature of the second particulate coating layer may be evident when the coating is visually examined macroscopically. In one embodiment, when the coating surface is analyzed using microscopy techniques such as scanning electron microscopy (SEM) at a suitable magnification e.g. 5000x, an abundance of individual particles of roughly 1 µm length can be observed.

In general, the release of excess particulates from the coatings of intracorporeal medical devices is to be avoided, since the release *in vivo* of, in particular, polymer particles can pose a health risk for patients (e.g. inflammation and emboli). However, these potential problems are usually associated with the release of e.g. polymer particles of diameter > 10 µm, which are insoluble or sparingly soluble in the blood stream. The second particulate coating layers described herein are composed of soluble, typically non-polymer based particles to be delivered to a treatment site, therefore the release of such particles when the paclitaxel coating elutes from the surface of the device will have only a therapeutic effect (associated with the paclitaxel) and none of the adverse effects mentioned above.

In one embodiment, the paclitaxel and the or each organic additive are in crystalline form.

In one embodiment of the invention, a characteristic of the second particulate coating layer comprising paclitaxel and at least one organic additive is that at least a proportion of the second particulate layer exhibits a depressed melting endotherm. A melting endotherm can be observed in a differential scanning calorimetry (DSC) measurement, as described in Example 4. Thus, "melting point" and "peak melting endotherm" as referred to herein should be considered as being equivalent. A "depressed melting endotherm" is observed when a proportion of the second particulate coating layer comprising paclitaxel and at least one organic additive melts as a single phase at a lower temperature than the melting point of either paclitaxel or the at least one organic additive when in pure form. If the second particulate coating layer contains more than one organic additive, the depressed melting endotherm is lower than the melting points of all of the organic additives present in the second layer.

Thus, in one embodiment at least a proportion of the second particulate coating layer comprising paclitaxel and at least one organic additive melts as a single phase at a lower temperature than the melting point of paclitaxel and the at least one organic additive in pure form.

Reference to "at least a proportion" of the second particulate coating layer exhibiting a depressed melting point is intended to cover the scenario when the entire second particulate coating layer comprising paclitaxel and at least one organic additive exhibits a depressed melting point i.e. the remaining portion of the second particulate coating layer (other than the defined "proportion") can also exhibit the same depressed melting point.

The proportion of the second particulate coating layer comprising paclitaxel and the at least one organic additive which exhibits a depressed melting point melts at the lower temperature as a single phase i.e. a single depressed melting point is observed at which point both the paclitaxel and the at least one organic additive melt simultaneously.

In some embodiments, the second layer particulate coating layer comprising paclitaxel and the at least one organic additive is predominantly in crystalline form.

The second particulate coating layer may comprise crystalline particles of paclitaxel and the at least one organic additive in the form of a eutectic mixture, wherein the eutectic mixture exhibits a depressed melting point. A eutectic mixture is defined herein as an intimately blended physical mixture of two or more components (suitably wherein at least one and preferably all of the components are crystalline) which melts as a single phase having a melting point lower than that of either or any of its components. A eutectic mixture tends to form when the two (or more) different crystalline components are mismatched in terms of molecular size or molecular shape such that cohesive interactions are relatively stronger than adhesive interactions, leading to a conglomerate of the two or more lattice structures, rather than a new lattice structure. Therefore, an X-ray powder diffraction ("XRPD") pattern of such a paclitaxel-organic additive coating would be expected to have an XRPD pattern identical to, or substantially similar to, a superimposition of the individual XRPD patterns of paclitaxel and the organic additive. The XRPD pattern of such a coating would not have a unique lattice arrangement distinct from the individual components therefore peaks other than those corresponding to the paclitaxel and organic additive would not be visible (Cherukuvada et al., 2014, Chem. Comm, Vol. 50, pages 906-923). Without wishing to be bound by theory, the inventors believe that the colligative properties and high thermodynamic functions (e.g. free energy, enthalpy and entropy) of a eutectic drug coating composition could allow rapid transfer of the drug from the coating to an adjacent tissue, while minimizing the nonspecific loss of drug from the coating prior to transfer to the adjacent tissue.

Alternatively, the second particulate coating layer may comprise particles of crystalline material comprising paclitaxel and the at least one organic additive, sometimes referred to as a "co-crystal", wherein the crystalline material exhibits a depressed melting point. A co-crystal rather than a eutectic system is more likely to be formed when the two (or more) individual components have a strong adhesive interaction leading to an essentially single continuous crystalline phase. A co-crystalline paclitaxel-organic additive second layer could therefore be expected to exhibit a unique XRPD pattern, different from that of the paclitaxel or organic additive (Cherukuvada et al., 2014, Chem. Comm, Vol. 50, pages 906-923).

It is widely accepted that there are no ground rules or structural guidelines as to the point at which the cohesive interactions dominate over the adhesive interactions (to give a eutectic) and *vice versa* (to give a co-crystal). It should be noted that the exact structural nature of the second particulate coating layer (i.e. eutectic, co-crystal or mixture thereof) is not required to be understood for the working of the present invention, even in those embodiments wherein the second particulate coating layer exhibits a depressed melting point.

In this particular embodiment, the relative amounts of paclitaxel and the at least one organic additive in the second particulate coating layer should be such that at least a proportion of the second particulate coating layer will exhibit a depressed melting point. This will depend to some extent on the nature of the at least one organic additives, but can easily be determined by varying the ratio of the two components and analysing the resulting second particulate coating layer coatings by DSC to determine whether the depressed melting point is present.

The second particulate coating layer can be analysed independently of the first coating layer by scraping a sample of the second particulate coating layer off a coated device of the invention (see Example 4). Alternatively, where a non-solid analysis can be performed, the second particulate coating layer can be extracted by immersing the implantable medical device in a suitable solvent, e.g. 0.2% acetic acid in methanol, such that the second particulate coating layer dissolves into the solution, leaving the first coating layer intact on the surface of the medical device (see Test Method C-II).

In one embodiment, substantially all of the second particulate coating layer comprising paclitaxel and the at least one organic additive melts as a single phase at a lower temperature than the melting point of either the paclitaxel or the at least one organic additive when in pure form. In this embodiment, a DSC thermogram of the second particulate coating layer would be expected to show a single depressed melting point and no visible endotherm corresponding to the melting of pure paclitaxel or at least one pure organic additive.

In one embodiment, 20-100% (by weight) of the second particulate coating layer exhibits a depressed melting point (i.e. a melting point which is at a lower temperature than the melting point of the paclitaxel and the at least one organic additive in pure form) such as 30-100%, 40-100%, 50-100%, 60-100%, 70-100%, 80-100% 90-100% or substantially all of the second particulate coating layer exhibits a depressed melting point. In embodiments where less than 100% of the second particulate coating layer is in a form which exhibits a depressed melting point, the remaining material will be paclitaxel in pure form, or at least one (if present) of the organic additives in pure form, or a mixture thereof.

In one embodiment, a proportion of the second particulate coating layer comprising paclitaxel and at least one organic additive melts as a single phase at a lower temperature than the melting point of either the paclitaxel or the at least one organic additive when in pure form, and the remaining second particulate coating layer comprising paclitaxel and at least one organic additive melts at or close to the melting point of the at least one organic additive in pure form. In this embodiment, a DSC thermogram of the second particulate coating layer would be expected show a single depressed melting point and an endotherm at or close to the known melting point for the at least one pure organic additive. In one embodiment, "close to" the known melting point means within ±10 °C of the known melting point for the pure organic additive, for example within ±5 °C, within ±4 °C, within ±3 °C, within ±2 °C or within ±1 °C. In this embodiment, the proportion of organic additive which melts at a temperature at or close to the melting point of the organic additive in pure form is suitably lower than the proportion of organic additive in the paclitaxel-organic additive material which melts with a single depressed melting point. In a second particulate coating layer comprising two organic additives, a DSC thermogram may show a single depressed melting point and one or two endotherms corresponding to the known melting point of one, or both of the organic additives in pure form.

In an embodiment wherein the second particulate layer exhibits a depressed melting point, the proportion of organic additive which melts at a temperature at or close to the melting point of the organic additive in pure form is 1-80% (wt%) of the organic additive in the second particulate coating layer e.g. 1-70%, 1-60%, 1-50%, 1-40%, 1-30%, 1-20%, 1-10%, 1-5% or 1-2%.

In a further embodiment wherein the second particulate layer exhibits a depressed melting point, a proportion of the second particulate coating layer comprising paclitaxel and at least one organic additive melts as a single phase at a lower temperature than the melting point of either the paclitaxel or the at least one organic additive when in pure form, and the remaining second particulate coating layer comprising paclitaxel and at least one organic additive melts at or close to the melting point of the paclitaxel in pure form. In this embodiment, a DSC thermogram of the second particulate coating layer will show a single depressed melting point and an endotherm at or close to the known melting point for paclitaxel. In this embodiment, the proportion of paclitaxel which melts at a temperature at or close to the melting point of the paclitaxel in pure form is suitably lower than the proportion of paclitaxel in the paclitaxel-organic additive material which melts with a single depressed melting point.

In one embodiment, the proportion of paclitaxel which melts at a temperature at or close to the melting point of the paclitaxel in pure form is 1-80% (wt%) of the paclitaxel in the second particulate coating layer e.g. 1-70%, 1-60%, 1-50%, 1-40%, 1-30%, 1-20%, 1-10%, 1-5% or 1-2%.

In a still further embodiment, a proportion of the second particulate coating layer comprising paclitaxel and at least one organic additive melts as a single phase at a lower temperature than the melting point of either the paclitaxel or the at least one organic additive when in pure form, and the remaining second particulate coating layer comprising paclitaxel and at least one organic additive exhibits two melting endotherms: one at or close to the melting point of the paclitaxel in pure form and the other at or close to the melting point of the at least one organic additive in pure form. In this embodiment, a DSC thermogram of the second particulate coating layer will show a single depressed melting point and one endotherm at or close to the known melting point for paclitaxel and another endotherm at or close to the known melting point for the at least one organic additive. In this embodiment, the proportion of paclitaxel which melts at a temperature at or close to the melting point of the paclitaxel in pure form is suitably less that the proportion of paclitaxel in the paclitaxel-organic additive material which melts with a single depressed melting point, and the proportion of organic additive which melts at a temperature at or close to the melting point of the at least one organic additive in pure form is suitably less that the proportion of organic additive in the paclitaxel-organic additive material which melts with a single depressed melting point.

The relative proportions of 1) paclitaxel/organic additive composition exhibiting a depressed melting point; and 2) paclitaxel/organic additive composition with a melting point at or close to the melting point of pure paclitaxel and/or organic additive can be determined by DSC analysis because the area under the relevant endotherms can be correlated to the relative amount of each component 1) or 2) in the second particulate coating layer as a whole (in terms of weight, which can be converted to a molar ratio if required).

As mentioned above, the second particulate coating layer can be analysed by ultra-performance liquid chromatography (UPLC - see Test Method C-II and Evaluation Methods) and/or by mass spectrometry to determine the amount of paclitaxel in the second particulate coating layer. When the weight % of paclitaxel in the second particulate coating layer is known, as in the case of a binary second particulate coating layer (i.e. paclitaxel + one organic additive only) then the weight % of the organic additive can easily be determined as being 100 - paclitaxel wt%.

In one embodiment, the weight % of paclitaxel in the second particulate coating layer is between about 5 wt. % and about 95 wt. %, for example between about 10 wt. % and about 95 wt. %, between about 20 wt. % and about 95 wt. %, between about 30 wt. % and about 90 wt. %, between about 45 wt. % and about 85 wt. %, between about 55 wt. % and about 70 wt. %, between about 40 wt. % and about 80 wt. %, between about 25 wt % and about 95 wt.%, between about 30 wt.% and about 85 wt. %, between about 70 wt. % and about 95 wt. %, 70 wt. % and about 80 wt. % or between about 75 wt. % and about 80 wt. %.

In one embodiment, the organic additive is caffeine and the weight % of paclitaxel in the second particulate coating layer is between about 70 wt. % and about 95 wt. %, for example between about 75 wt. % and about 90 wt. %. In one embodiment, the organic additive is caffeine and the ratio (wt. %) of paclitaxel: caffeine in the second particulate coating layer is between about 7:3 and about 95:5, for example between about 3:1 and about 9:1 wt. %.

In one embodiment, the organic additive is succinic acid and the weight % of paclitaxel in the second particulate coating layer is between about 70 wt. % and about 90 wt. %, for example between about 75 wt. % and about 85 wt. %. In one embodiment, the organic additive is succinic acid and the ratio (wt. %) of paclitaxel:succinic acid in the second particulate coating layer is between about 7:3 and about 9:1, for example between about 3:1 wt. % and about 6:1.

In one embodiment the organic additive is selected from the group consisting of p-aminobenzoic acid (PABA), saccharin, ascorbic acid, methyl paraben, caffeine, calcium salicylate, pentetic acid, creatinine, ethylurea, acetaminophen, aspirin, theobromine, tryptophan, succinic acid, glutaric acid, adipic acid, theophylline, and saccharin sodium, and weight % of paclitaxel in the second particulate coating layer is between about 30 wt. % and about 90 wt. %, such as between about 50 wt. % and about 90 wt. %.

In one embodiment the organic additive is selected from the group consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, glutaric acid, adipic acid, theophylline, and saccharin sodium, and the ratio (wt. %) of paclitaxel: organic additive in the second particulate coating layer is between about 3:7 and about 9:1, such as between about 1:1 and about 9:1.

In one embodiment the organic additive is selected from the group consisting of p-aminobenzoic acid, methyl paraben, caffeine, calcium salicylate and succinic acid, and the weight % of paclitaxel in the second particulate coating layer is between about 30 wt. % and about 90 wt. %, such as between about 50 wt. % and about 90 wt. %.

In one embodiment the organic additive is selected from the list consisting of p-aminobenzoic acid, methyl paraben, caffeine, calcium salicylate and succinic acid and the ratio (wt. %) of paclitaxel:organic additive in the second particulate coating layer is between about 3:7 and about 9:1, such as between about 1:1 and about 9:1.

The second particulate coating layer can be prepared by a multitude of methods. One method of preparing the second particulate coating layer is by evaporation of a solution of paclitaxel and the at least one organic additive, which is applied to the surface to be coated.

Thus, in one aspect of the invention is provided an implantable medical device as described herein wherein the second particulate coating layer is applied to the medical device by dissolving the paclitaxel and the at least one organic additive in a solvent to form a solution, applying the solution to the medical device (i.e. the surface of the medical device to be coated, which is suitably pre-coated with the first coating layer) coating the device with the solution and evaporating the solvent.

Various methods for forming the second particulate coating layer by evaporation of a solution of paclitaxel and at least one organic additive can be used. The solution of paclitaxel and at least one organic additive can be pipetted over the exterior surface of the device, which is itself under rotation, e.g. pipetting 90-100 ul of the coating solution over the device at a time. Alternatively, the device can simply be dipped into the solution of paclitaxel and at least one organic additive, removed and then air dried. The dipping and drying process can be repeated as many times as is necessary to achieve the desired coating thickness or loading of paclitaxel. Representative procedures are described in Examples 2, 3, 3a, 3b and 3c. Other techniques such as casting, spinning, spraying, inkjet printing, electrostatic techniques, painting, dispersion coating, powder coating, or combinations thereof may be used to form the coating.

Suitably the second particulate coating layer is applied by dispensing the coating solution comprising paclitaxel, organic additive and solvent on to the surface of the device to be coated using a syringe pump, under rotation. Using this method, when a known volume and concentration of solution is coated on to a device (via "drop casting") the amount or loading of paclitaxel applied in the coating can be estimated, as set out in Example 3.

Suitably the solution of the paclitaxel and at least one organic additive is a solution in a solvent selected from water, acetone and mixtures thereof, for example between about 50/50 and about 95/5, between about 60/40 and about 90/10, between about 70/30 and about 90/10 or between about 70/30 and about 75/25 acetone/water (v/v) such as 90/10, 75/25, or 70/30 acetone/water (v/v).

Following application of the coating solution a drying step may be required. The coating drying environment may be controlled as a function of time, such as by controlling/modulating the air composition, flow rate and flow patterns, air temperature, localized heating (e.g., heat lamp), etc. to thereby control physical properties of the coating.

Thus, in one embodiment the second particulate coating layer is formed by a process comprising the steps of:
A) dissolving the paclitaxel and the at least one organic additive in a solvent to form a solution; and then
B) applying the solution to the implantable medical device; and then
C) evaporating the solvent.

Optionally the process includes additional step D) drying the coating.

A method of coating a stent with a first coating layer and a second particulate coating layer of paclitaxel and caffeine is described in Example 2. Methods of coating a stent-graft (pre-coated with a first coating layer) with second particulate coating layers containing paclitaxel and caffeine or succinic acid are described in Examples 3, 3a, 3b and 3c, each of which utilise different paclitaxel loadings (500 µg, 25 µg and 150 µg, respectively). These methods can be adapted for other implantable devices.

In one embodiment, the or an organic additive is caffeine and the weight % of paclitaxel in the pipetting/dipping solution (based on the total weight of solid components added) is between about 70 wt. % and about 95 wt. %, for example between about 75 wt. % and about 90 wt. %. In one embodiment, the or an organic additive is caffeine and the ratio (wt. %) of paclitaxel: caffeine in the pipetting/dipping solution (based on the total weight of solid components added) is between about 7:3 and about 95:5, for example between about 3:1 and about 9:1 wt. %.

In one embodiment, the or an organic additive is caffeine and the ratio (wt. %) of paclitaxel: caffeine in the pipetting/dipping solution (based on the total weight of solid components added) is between about 7:3 and about 95:5, for example between about 3:1 and about 9:1, wherein the dipping/pipetting solution is a solution of between about 70/30 and about 90/10 acetone/water (v/v).

In one embodiment, the or an organic additive is succinic acid and the weight % of paclitaxel in the pipetting/dipping solution (based on the total weight of solid components added) is between about 70 wt. % and about 90 wt. %, for example between about 75 wt. % and about 85 wt. %. In one embodiment, the or an organic additive is succinic acid and the ratio (wt. %) of paclitaxel:succinic acid in the pipetting/dipping solution (based on the total weight of solid components added) is between about 7:3 and about 9:1, for example between about 3:1 wt. % and about 6:1.

In one embodiment, the or an organic additive is succinic acid and the ratio (wt. %) of paclitaxel:succinic acid in the pipetting/dipping solution (based on the total weight of solid components added) is between about 7:3 and about 9:1, for example between about 3:1 wt. % and about 6:1, wherein the dipping/pipetting solution is a solution of between about 70/30 and about 90/10 acetone/water (v/v).

In one embodiment the at least one organic additive is independently selected from the group consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, glutaric acid, adipic acid, theophylline, and saccharin sodium, and the weight % of paclitaxel in the pipetting/dipping solution (based on the total weight of solid components added) is between about 30 wt. % and about 90 wt. %, such as between about 50 wt. % and about 90 wt. %.

In one embodiment the at least one organic additive is independently selected from the group consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, glutaric acid, adipic acid, theophylline, and saccharin sodium, and the ratio (wt. %) of paclitaxel:organic additive (total) in the pipetting/dipping solution (based on the total weight of solid components added) is between about 3:7 and about 9:1, such as between about 1:1 and about 9:1.

In one embodiment the at least one organic additive is independently selected from the group consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, glutaric acid, adipic acid, theophylline, and saccharin sodium, and the ratio (wt. %) of paclitaxel:organic additive (total) in the pipetting/dipping solution (based on the total weight of solid components added) is between about 3:7 and about 9:1, such as between about 1:1 and about 9:1, wherein the dipping/pipetting solution is a solution of between about 70/30 and about 90/10 acetone/water (v/v).

In one embodiment, the at least one organic additive is independently selected from the list consisting of p-aminobenzoic acid, methyl paraben, caffeine, calcium salicylate and succinic acid and the weight % of paclitaxel in the pipetting/dipping solution (based on the total weight of solid components added) is between about 30 wt. % and about 90 wt. %, such as between about 50 wt. % and about 90 wt. %.

In one embodiment, the at least one organic additive is independently selected from the list consisting of p-aminobenzoic acid, methyl paraben, caffeine, calcium salicylate and succinic acid and the ratio (wt. %) of paclitaxel:organic additive (total) in the pipetting/dipping solution (based on the total weight of solid components added) is between about 3:7 and about 9:1, such as between about 1:1 and about 9:1.

In one embodiment, the at least one organic additive is independently selected from the list consisting of p-aminobenzoic acid, methyl paraben, caffeine, calcium salicylate and succinic acid and the ratio (wt. %) of paclitaxel: organic additive (total) in the pipetting/dipping solution (based on the total weight of solid components added) is between about 3:7 and about 9:1, such as between about 1:1 and about 9:1, wherein the dipping/pipetting solution is a solution of between about 70/30 and about 90/10 acetone/water (v/v).

In one embodiment, the organic additive is caffeine and the ratio (wt. %) of paclitaxel:caffeine in the pipetting/dipping solution (based on the total weight of solid components added) is between about 3:1 and about 6:1, wherein the dipping/pipetting solution is a solution of between about 70/30 and about 90/10 acetone/water (v/v).

In one embodiment, the organic additive is succinic acid and the ratio (wt. %) of paclitaxel:succinic acid in the pipetting/dipping solution (based on the total weight of solid components added) is between about 3:1 and about 6:1, wherein the dipping/pipetting solution is a solution of between about 70/30 and about 90/10 acetone/water (v/v).

Typically, the second particulate coating layer will have an average total thickness of about 0.1 µm to about 200 µm, such as about 0.2 µm to about 100 µm. Coating thickness can be measured using a suitable coating thickness analyser or gauge, or by using X-ray photoelectron spectroscopy with depth profiling (see Evaluation Methods).

Alternatively, the second particulate coating layer may be applied to the implantable medical device using a method which involves minimal solvent, or indeed no solvent. For example, a dry powder method may be used which involves combining the paclitaxel and at least one organic additive in powder form before applying to the device to form a solid particulate composition, optionally followed by thermal treatment. The powder mixture of paclitaxel and at least one organic additive is suitably spayed on to the device, which optionally comprises an adhesive layer (as described hereinabove), which may be followed by thermal treatment, for example, to affix the layer to the surface of the device (which is suitably pre-coated with the first coating layer).

Thus, in one embodiment the second particulate coating layer is formed by a process comprising the steps of combining the paclitaxel and the at least one organic additive in powder form, and then applying the powder to the implantable medical device (which is suitably pre-coated with the first coating layer) to form a solid particulate composition. An additional thermal treatment step may subsequently be applied, for example to affix the coating to the surface of the medical device.

### Additional coating layers

The first coating layer is typically applied to the implantable medical device before the second particulate coating layer. However, the first coating layer need not be applied directly to a surface of the implantable medical device. The implantable medical device can also include additional coatings underlying or overlying the first coating layer and second particulate coating layer. Such additional coatings are separate and distinct from the first coating layer and the second particulate coating layer and provide additional benefits while maintaining the heparin moiety bioactivity of the first coating layer and the ability of the paclitaxel to elute from the second particulate coating layer. These additional coatings can include other therapeutic agents, alone or in combination with various excipients or carriers. In one embodiment, the amount or thickness of the additional coating may be varied over the surface of the implantable medical device. The additional coating layer can be continuous over an entire surface of the device or be discontinuous and cover only a portion or separate portions of the device. The additional coating layer can also be "sculpted" or modified to create a desired surface topography or texture.

In one embodiment, an adherent layer is interposed between the first coating layer and the material of the surface of the implantable medical device. The adherent layer, which is a separate and distinct layer underlying the first coating layer and second particulate coating layer improves the adherence of the first coating layer to the surface of the implantable medical device and further maintains the integrity of the coating, particularly during transit to the tissue to the be treated. In one embodiment, the adherent layer comprises a polymer, which is suitably biocompatible and avoids irritation of body tissue. Examples of such polymers include, but are not limited to polyolefins, polyisobutylene, ethylene-α-olefin copolymers, acrylic polymers and copolymers, polyvinyl chloride, polyvinyl methyl ether, polyvinylidene fluoride and polyvinylidene chloride, fluoropolymers, e.g. expanded polytetrafluoroethylene (ePTFE), polytetrafluoroethylene (PTFE), fluorinated ethylene-propylene (FEP), perfluorocarbon copolymers, e.g. tetrafluoroethylene perfluoroalkylvinyl ether (TFE/PAVE) copolymers, copolymers of tetrafluoroethylene (TFE) and perfluoromethyl vinyl ether (PMVE), copolymers of TFE with functional monomers that comprise acetate, alcohol, amine, amide, sulfonate, functional groups and the like as described in U.S. Pat. No. 8,658,707 (W.L. Gore and Associates, as well as combinations thereof), polyacrylonitrile, polyvinyl ketones, polystyrene, polyvinyl acetate, ethylene-methyl methacrylate copolymers, acrylonitrile-styrene copolymers, ABS resins, Nylon 12 and its block copolymers, polycaprolactone, polyoxymethylenes, polyethers, epoxy resins, polyurethanes, rayon-triacetate, cellulose, cellulose acetate, cellulose butyrate, cellophane, cellulose nitrate, cellulose propionate, cellulose ethers, carboxymethyl cellulose, chitins, polylactic acid, polyglycolic acid, polylactic acid-polyethylene oxide copolymers, polyethylene glycol, polypropylene glycol, polyvinyl alcohol, elastomeric polymers such as silicones (e.g., polysiloxanes and substituted polysiloxanes), polyurethanes, thermoplastic elastomers, ethylene vinyl acetate copolymers, polyolefin elastomers, EPDM rubbers and mixtures thereof.

An additional adherent layer between the surface of the device and the first coating layer would be selected by the skilled person on the basis that it would not impact the heparin moiety bioactivity of the subsequently-applied first coating layer. Heparin bioactivity can be assessed following, for example, Test Method J or K.

In another embodiment, an additional coating layer comprising a therapeutic agent other than paclitaxel is interposed between the first coating layer and the material of the surface of the implantable medical device, or is applied to at least a portion of the first coating layer and/or second particulate coating layer. Said additional coating layer is a layer which is separate and distinct from the first coating layer and second particulate coating layer and may provide a therapeutic benefit in addition to the benefit provided by the paclitaxel and heparin moiety i.e. allowing for adjunctive therapies to be combined with the paclitaxel-organic additive and heparin moiety.

In one embodiment, the implantable medical device further comprises a protective top coat overlying the surface of the second particulate coating layer, or the first coating layer and the second particulate coating layer. The top coat may further minimise loss of the coating layer, in particular the second particulate coating layer (paclitaxel-organic additive layer) before it is brought into contact with target tissues, for example during device assembly and packaging, transit to the site to be treated, or if the device is a stent or stent-graft, during the first moments of expansion before second particulate coating layer is pressed into direct contact with target tissue.

In embodiments wherein the implantable medical device of the invention comprises an additional coating layer, such additional layers are considered to be distinct and separate layers to the first coating layer comprising an immobilized heparin moiety and the second particulate coating layer which comprises paclitaxel and at least one organic additive. For example, while in certain embodiments of the invention the second particulate coating layer is defined as being surfactant free and/or polymer free, the implantable medical device can still have a distinct and separate coating layer comprising surfactant and/or polymer, either underlying or overlying the first coating layer and/or second particulate coating layer, or residing in between a portion of the first coating layer and second particulate coating layer. Similarly, although in one embodiment the second particulate coating layer does not contain protein, the implantable medical device may have a further coating layer, underlying or overlying the first coating layer and/or second particulate coating layer, or residing in between a portion of the first coating layer and second particulate coating layer, which comprises protein. Thus, a component in the additional coating layer will not form part of first coating layer or second particulate coating layer.

In a situation where a medical device has multiple coating layers in addition to the coating layer of the invention, in embodiments where the second particulate coating layer exhibits a depressed melting point, this may be difficult to confirm. However, in this situation, the presence of a melting point which does not correspond to any of the coating components is suggestive of the formation of paclitaxel-organic excipient material exhibiting a depressed melting point, particularly if the characteristic melting endotherms corresponding to paclitaxel and the organic excipient are also absent.

Any additional coating layers (in particular an additional top coating) should still allow the paclitaxel to elute from the second particulate coating layer, once in contact with the target tissue. The rate of elution of paclitaxel from a device can be assessed following Test Method M. The bioactivity of the heparin moiety of the first coating layer should also not be adversely affected in a significant way by the application of any additional coatings.

In one embodiment, the implantable medical device has a coating consisting of a first coating layer and a second particulate coating layer as described herein i.e. the device has no additional coating layers.

Prior to applying the first coating layer the surface of the implantable medical device may be cleaned to improve adhesion and surface coverage. Suitable cleaning agents include solvents as ethanol or isopropanol (IPA), solutions with high pH like solutions comprising a mixture of an alcohol and an aqueous solution of a hydroxide compound (e.g. sodium hydroxide), sodium hydroxide solution as such, solutions containing tetramethyl ammonium hydroxide (TMAH), acidic solutions like Piranha (a mixture of sulfuric acid and hydrogen peroxide ), and other oxidizing agents including combinations of sulfuric acid and potassium permanganate or different types of peroxysulfuric acid or peroxydisulfuric acid solutions (also as ammonium, sodium, and potassium salts).

### Composition and properties of coating layers

The first coating layer and second particulate coating layer are applied to a surface of an implantable medical device such that at least a portion of the second particulate coating layer is in contact with at least a portion of the first coating layer.

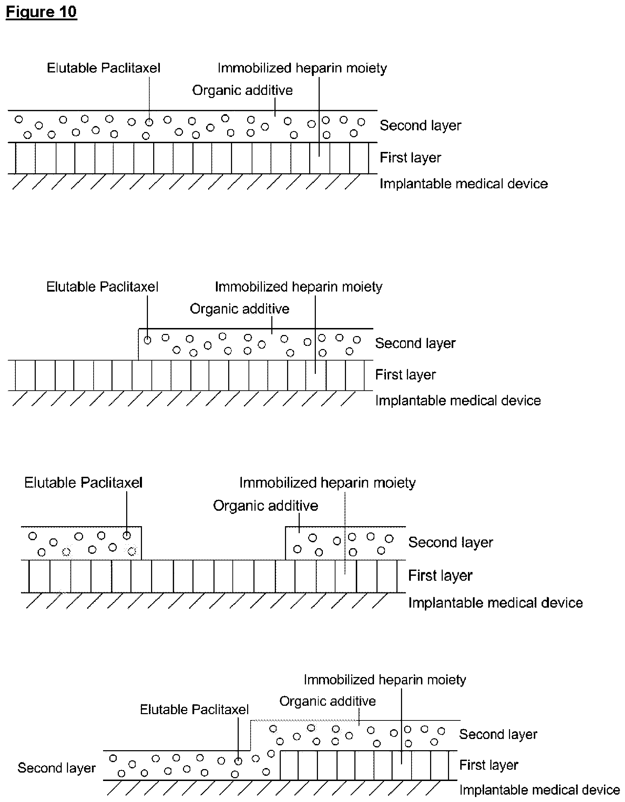

The first coating layer is applied to a surface of the implantable medical device before application of the second particulate coating layer, and preferably the second particulate coating layer is applied to a surface of the implantable medical device already covered with the first coating layer i.e. preferably the second particulate coating is applied on top of at least a portion of the first coating layer (as illustrated in Figure 10).

Thus, the present invention provides a process for preparing a coated implantable medical device comprising the steps of:
i) treating the medical device to provide a first coating layer comprising an immobilized heparin moiety; and further
ii) treating the medical device to provide a second particulate coating layer comprising elutable paclitaxel and at least one organic additive,
wherein at least a portion of the second particulate coating layer is in contact with at least a portion of the first coating layer.

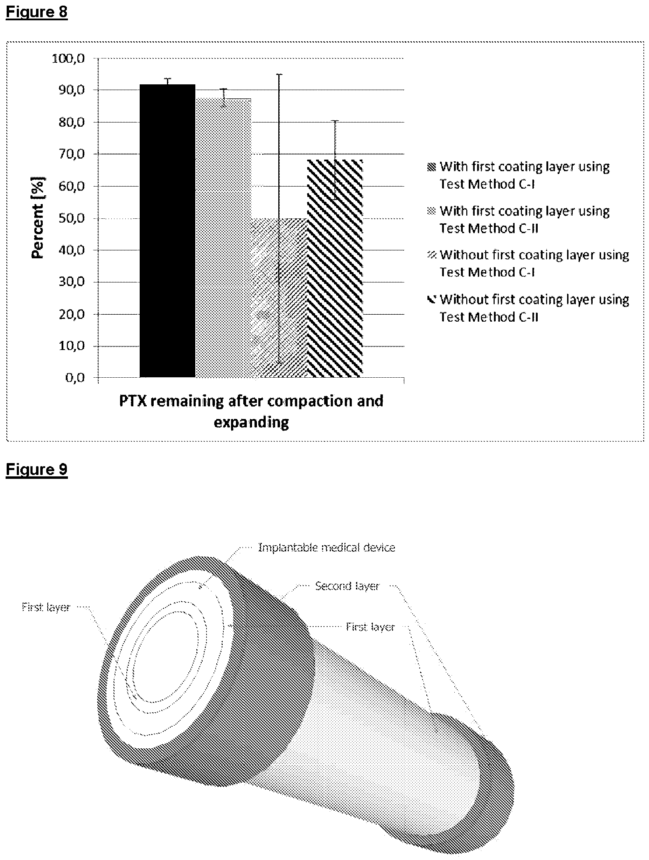

In one embodiment, the first coating layer is applied to the medical device before the second particulate coating layer. In another embodiment, at least a portion of the second particulate coating layer is applied on top of at least a portion of the first coating layer (see for example Figures 9 and 10, showing some possible arrangements of the first coating layer and second particulate coating layer which would fall under this embodiment).

Embodiments described separately above with respect to preparing the first coating layer (step i)) and the second particulate coating layer (step ii)) apply equally to the embodiments describing the overall coating process (i.e. steps i) and ii)). Embodiments described above with respect to the composition of the first coating layer and second coating layer apply equally to all process embodiments.

The paclitaxel is "elutable" in the sense that it is released from the coating layer of the implantable medical device (specifically from the second particulate coating layer) when implanted at the target area, i.e. the area to be treated with the paclitaxel. Elution of the paclitaxel results from degradation and/or dissipation of the second particulate coating layer i.e. once the paclitaxel has eluted, the second particulate coating layer no longer exists. It should be noted that degradation in this context refers to the degradation of the coating structure as a whole, rather than chemical degradation of the coating components.

In one embodiment, the paclitaxel is considered to be elutable if when immersed 0.2% acetic acid in methanol, the second particulate coating layer dissolves completely into the solution, within 15 minutes, as described in Test Method C-II.

As discussed above, a particular challenge when developing an elutable drug coating for a medical device is to achieve a balance between having sufficient adhesion to the device such that the coating is not lost/damaged in transit, yet also having suitable release characteristics such that the drug will transfer from the coating to the target tissue (elute) i.e. if the adhesion of the coating is too strong, the coating will be durable but an insufficient amount of the drug will be released and will result in suboptimal efficacy. Conversely, a coating may have excellent release characteristics but if the coating does not have sufficient adhesion to the device then an insufficient amount of drug will reach the target tissue, and unintentional release of the drug in areas other than the target tissue may be detrimental to the patient.

The second particulate coating layer of the present invention provides a good balance of good adhesion to an implantable medical device, thereby minimising or even eliminating coating loss during manipulation of the device, and suitable release characteristics such that the paclitaxel is delivered in an effective and efficient manner to the target tissue.

The present inventors have found that the nature of the first coating layer comprising an immobilized heparin moiety (to which at least a portion of the second particulate coating layer is in contact) also impacts the adherence and release characteristics of the second particulate coating layer.

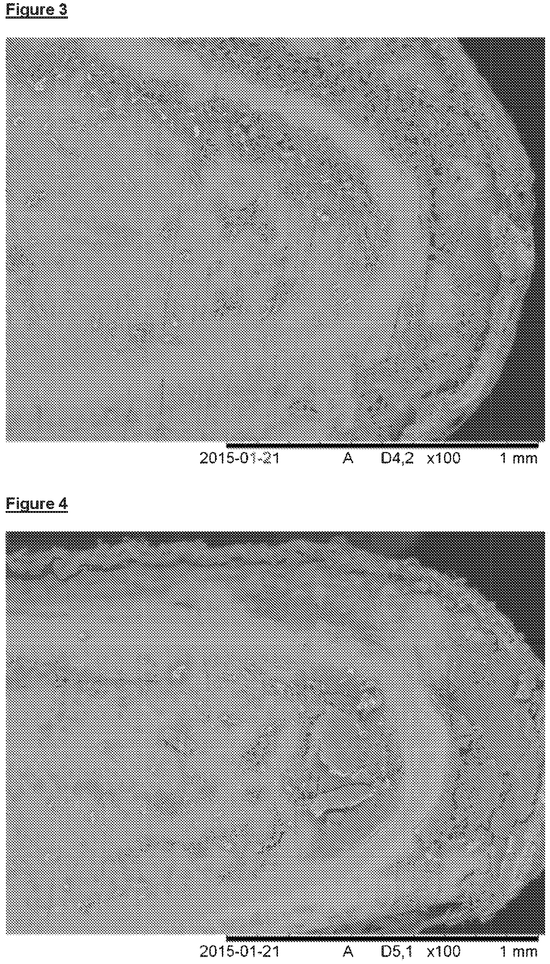

As shown in Figures 3 and 4 (Example 21) devices of the invention have a smooth and even outer coating layer which is maintained even following manipulation. This even coating layer has been attributed at least in part to the wettability of the immobilized heparin of the first coating layer (to which at least a portion of the second particulate coating layer is in contact), which is illustrated in Figure 5 (Example 23).

The paclitaxel release profiles of devices of the invention were investigated in Example 10. The highest release rate was observed in the initial period up the first time point (0.25 h) with even and sustained (lower) release rates being observed over the remaining period of observation (up to 24 h). Sterilization did not significantly impact the release profile, as observed in Example 11.

The durability of the coated stent prepared in Example 2 was assessed as described in Example 6 by comparing the mass of the coating before and after compaction and expansion. The coated stent was found to have a high degree of durability. The durability of the coated stent-grafts prepared according to Example 3, 3a, 3b and 3c were assessed via a different method described in Example 7, wherein the paclitaxel content on the device was measured before and after compaction and expansion. The coated stent-grafts were also found to have a high degree of durability.

In the present Examples the paclitaxel-organic additive layer (second particulate coating layer) is applied on top of a first coating layer composed of heparin immobilized onto a polyamine surface. As discussed above, paclitaxel is known to be unstable in the presence of amine functionality, therefore the fact that the paclitaxel content is essentially un-degraded following coating onto the first coating layer and subsequent storage and manipulation is surprising (see e.g. Example 7, indicating good retention of paclitaxel following manipulation).

In one embodiment, the implantable medical device is a stent and the second particulate coating layer has suitable adherence such that less than 40 % of the paclitaxel (wt%) is lost during manipulation using Test Method H-I followed by Test Method H-II, for example less than 30 %, less than 25 %, less than 20 %, less than 15 %, less than 10 % or less than 5 %, as determined using Test Method C-I or C-II.

In one embodiment, the implantable medical device is a stent-graft and the second particulate coating layer has suitable adherence such that less than 40 % of the paclitaxel (wt%) is lost during manipulation using Test Method I-I followed by Test Method I-II, for example less than 30 %, less than 25 %, less than 20 %, less than 15 %, less than 10 % or less than 5 %, as determined using Test Method C-I or C-II.

The durability of the coated surface of a stent-graft of the invention is further shown in Figures 3 and 4, which are SEM images of the surface of a stent graft prepared according to Example 3c, before and after manipulation (Example 21).

Thus, implantable medical devices of the present invention are durable, while also having desirable paclitaxel release characteristics.

The topography of the surface of the medical device onto which the first coating layer, or first coating layer and second particulate coating layer are coated can further enhance the adhesion and release characteristics of the second particulate coating layer. The present inventors have observed that when the first coating layer and second particulate coating layers are coated onto a surface with a porous structure, such as ePFTE, the adherence and release characteristics are particularly favourable. Without wishing to be bound by theory, it is thought that when a surface with pores is coated with the first coating layer and second particulate coating layer, the paclitaxel-organic additive layer may be contained within the pores. This could have the effect of stabilizing the paclitaxel during processing of the device, thereby enhancing coating durability, and allowing the use of lower loadings of paclitaxel on the device to be used. As shown in Example 7, a stent-graft of the invention with a lower initial loading of paclitaxel was observed to lose a lower amount (wt %) of paclitaxel following manipulation of the device, compared with a higher loading. This could also have the effect of slowing the release of paclitaxel into the target tissue once the medical device is implanted, thereby providing prolonged release of paclitaxel from the device to the target tissue.

Thus, in embodiments wherein the surface of the device being coated is porous e.g. the surface is composed of ePTFE, or knitted, woven or braided polyester, the wettable nature of the immobilized heparin moiety first coating layer and the porous nature of the surface could provide an implantable medical device with particularly favourable adhesion and release characteristics.

The implantable medical device of the invention, in particular the second particulate coating layer of the device, has suitable paclitaxel release and tissue transfer characteristics such that the measured drug concentration in the tissue at the 24 hr timepoint (measured according to Test Method A) is at least 1 µg drug per g tissue (µg/g), for example at least 2.5 µg/g, at least 5 µg/g, at least 10 µg/g, at least 50 µg/g or at least 100 µg/g.

In one embodiment, the implantable medical device is a stent, and the second particulate coating layer of the device has suitable paclitaxel release and tissue transfer characteristics such that using Test Method A the measured drug concentration in the tissue at the 24 hr timepoint is at least 1 µg drug per g tissue (µg/g), for example at least 2.5 µg/g, at least 5 µg/g or at least 10 µg/g.

In one embodiment, the implantable medical device is a stent-graft, and the second particulate coating layer of the device has suitable paclitaxel release and tissue transfer characteristics such that using Test Method A the measured drug concentration in the tissue at the 24 hr timepoint is at least 1 µg drug per g tissue (µg/g), for example at least 10 µg/g, at least 50 µg/g or at least 100 µg/g.

The paclitaxel release characteristics may be further enhanced by considering the location of the first coating layer and second particulate coating layer on the implantable medical device.

Implantable medical devices suitably have an external surface and an internal surface, either or both of which can be coated with the first coating layer and second particulate coating layer.

For example, tubular substrates including but not limited to artificial blood vessels, vascular grafts, stents, and stent-grafts have an internal surface (luminal surface/side), which can be coated independently from the external surface (abluminal surface/side). A device comprising an internal and an external surface may only require the external surface to be coated. Conversely, only the internal surface may require a coating of the invention. Alternatively, both the internal and external surfaces may require coating, but with different coatings, or different combinations of coatings.

The amount or thickness of each coating layer may independently be varied over the surface of the medical device. Each coating layer can independently be continuous over an entire surface of the device or be discontinuous and cover only a portion or separate portions of the device (e.g. as illustrated in Figure 10).

In one embodiment, both the external and internal surfaces of the device are coated with the first coating layer. In another embodiment, only a portion of the external surface of the device is coated with the second particulate coating layer.

In one embodiment, the first coating layer is applied to up to 100%, for example up to 99%, 95%, 90%, 75%, 50% or 25% of the surface area of the implantable medical device.

In one embodiment the implantable medical device is a tubular implantable medical device with a luminal and an abluminal side. In this embodiment, suitably the first coating layer is applied to a portion of the luminal side and to a portion of the abluminal side of the medical device; and the second particulate coating layer is applied only to a portion of the abluminal side of the device. "A portion" as referred to herein should be taken to mean "at least a portion" such that the entire side may be coated.

In one embodiment, the implantable medical device is a tubular medical device and the first coating layer is applied to up to 100%, for example up to 99%, 95%, 90%, 75%, 50% or 25% of the luminal side and up to 100%, for example up to 99%, 95%, 90%, 75%, 50% or 25% of the abluminal side. Suitably the tubular medical device is a stent or stent-graft.

In one embodiment, the first coating layer is applied to the entire luminal side and abluminal side of the medical device. In one embodiment, the second particulate coating layer is applied to the entire abluminal side of the medical device.

In one embodiment, the second particulate coating layer is applied to up to 100%, for example up to 99%, 95%, 90%, 75%, 50% or 25% of the surface area of the implantable medical device.

In one embodiment the implantable medical device is a tubular implantable medical device wherein the second particulate coating layer is applied to up to 100%, for example up to 99%, 95%, 90%, 75%, 50% or 25% of the abluminal surface side of the implantable medical device.

In another embodiment, the implantable medical device is a stent-graft and the first coating layer is independently applied to up to 100%, for example up to 99%, 95%, 90%, 75%, 50% or 25% of the luminal and abluminal sides of a stent-graft, wherein the second particulate coating layer is applied to up to 95%, 90%, 75%, 50% or 25% of the abluminal side of the graft member, in particular the graft member directly covering, or in contact with, the stent member.

In one embodiment, the first coating layer is applied to the entire surface area (100%) of the implantable medical device, and is then entirely over-coated with the second particulate coating layer (i.e. the second coating layer is also applied to 100% of the surface are of the medical device, which has been pre-coated with the first coating layer).

In another embodiment, the implantable medical device is a stent or stent-graft and the first coating layer is applied to the entire luminal and abluminal sides of the device, and the second particulate coating layer is applied only up to 100%, for example up to 99%, 95%, 90%, 75%, 50% or 25% of the abluminal side of the device. In this embodiment, suitably the device is a stent-graft.

It should be noted that in the above embodiments and those that follow, the surface area does not take into account porosity considerations of a device composed of a porous material. If the surface of the device is porous, the effect of porosity on surface area is not considered. For example, the surface area of a cylindrical tubular ePTFE vascular graft (which is made of a porous material) comprising the inner surface of the tubular graft is calculated as it is for any cylindrical geometry as 2πrL: where r is the graft inner radius; L is the axial length; and π is the number pi.

In one embodiment, the second particulate coating layer is applied only to a portion of the abluminal side of the implantable medical device.

In one embodiment, the second particulate coating layer is applied only to one end of the abluminal side of the implantable medical device. If the device has designated proximal and distal ends, suitably in this embodiment the second particulate coating layer is applied only to the proximal end of the implantable medical device. The proximal end of a device can be designated by considering the flow of blood through the device once it is implanted. Once implanted, blood flows from the proximal end to the distal end of the device.

When the implantable medical device is a tubular medical device, such as a stent or stent-graft, in one embodiment the portion of the abluminal surface up to 1 to 20 mm from one end (the proximal end if designated) is coated with the second particulate coating layer, e.g. up to 2 to 10 mm, e.g. up to 3 to 7 mm. Where the end of the device is non-uniform, e.g. scalloped, then this distance is measured from the outermost point of the end to be coated.

Applying the second particulate coating layer only to the proximal end of the implantable medical device localizes the delivery of paclitaxel to the vascular tissue at the proximal end, but also allows migration of the paclitaxel further "downstream" i.e. along the length of the vessel between the proximal and distal ends of the device. In such an embodiment, a certain amount of migration of paclitaxel "upstream" might be observed.

In one embodiment, the second particulate coating layer is applied to both ends of the implantable medical device, in particular to both ends of the abluminal side of the implantable medical device.

Figure 9 illustrates a tubular implantable medical device with the first coating layer covering the luminal side and abluminal side of the device, and the second particulate coating layer covering both ends of the abluminal side of the device.

When the implantable medical device is a tubular medical device, such as a stent or stent-graft, in one embodiment the portion of the abluminal surfaces up to 1 to 20 mm from each end of the device are independently coated with the second particulate coating layer, e.g. up to 2 to 10 mm, e.g. up to 3 to 7 mm.

An immobilized heparin moiety will provide a biocompatible and antithrombotic effect when in contact with mammalian blood, e.g. when coated on the luminal side of an implantable medical device within the vasculature. Surprisingly, the first coating layer with an immobilized heparin moiety still provides an antithrombotic effect following processing of the medical device i.e. heparin moiety bioactivity is preserved during subsequent coating processes (application of the second particulate coating layer) as illustrated in Example 14. Given the sensitive nature of heparin moieties it could not be predicted that the medical device of the invention, in particular the first coating layer, would retain therapeutically useful heparin moiety bioactivity following implantation.

Heparin moiety bioactivity, in particular heparin bioactivity, can be quantified using various means, including by measuring the ability, or capacity, of the heparin moiety to bind a known quantity of antithrombin III (ATIII) (as described in Test Method K), or a known quantity of heparin cofactor II (HCII) (as described in Test Method J).

In one embodiment, the implantable medical device has ATIII binding activity of greater than 1 pmol/cm² of surface according to Test Method K, before implantation. In another embodiment, the implantable medical device has ATIII binding activity of at least 5 pmol/cm², such as at least 10 pmol/cm² of surface according to Test Method K. before implantation.

In one embodiment, the implantable medical device has HCII binding activity of greater than 1 pmol/cm² of surface according to Test Method J, before implantation. In another embodiment, the implantable medical device has HCII binding activity of at least 5 pmol/cm², such as at least 10 pmol/cm² of surface according to Test Method J, before implantation.

In one embodiment, the implantable medical device has ATIII binding activity of greater than 1 pmol/cm² of surface according to Test Method K, after elution of the paclitaxel. In another embodiment, the implantable medical device has ATIII binding activity of at least 5 pmol/cm², such as at least 10 pmol/cm² of surface according to Test Method K, after elution of the paclitaxel.

In one embodiment, the implantable medical device has HCII binding activity of greater than 1 pmol/cm² of surface according to Test Method J, after elution of the paclitaxel. In another embodiment, the implantable medical device has HCII binding activity of at least 5 pmol/cm², such as at least 10 pmol/cm² of surface according to Test Method J, after elution of the paclitaxel.

In one embodiment, the implantable medical device has ATIII binding activity of greater than 1 pmol/cm² of surface according to Test Method K, following removal of the second particulate coating layer according to Test Method C-II. In another embodiment, the implantable medical device has ATIII binding activity of at least 5 pmol/cm², such as at least 10 pmol/cm² of surface according to Test Method K, following removal of the second particulate coating layer according to Test Method C-II.

In one embodiment, the implantable medical device has HCII binding activity of greater than 1 pmol/cm² of surface according to Test Method J, following removal of the second particulate coating layer according to Test Method C-II. In another embodiment, the implantable medical device has HCII binding activity of at least 5 pmol/cm², such as at least 10 pmol/cm² of surface according to Test Method J, following removal of the second particulate coating layer according to Test Method C-II.

Heparin moiety bioactivity can also be demonstrated using a blood contact activation method, as described in Test Method B. As shown in Example 20, stent-grafts of the invention had similar preservation compared with a reference stent-graft with a first coating layer of immobilized heparin but no second particulate coating layer, indicating that application of the second particulate coating layer does not adversely affect in a significant way the thromboresistance of the first coating layer, which is surprising given the sensitive nature of heparin.

Furthermore, the application of the second particulate coating layer does not impact the uniform coverage of the first coating layer of immobilized heparin, as illustrated in Figure 5 which shows the staining of a stent-graft of the invention (right) compared with a reference stent graft with only a first coating layer (left) (Example 22).

Thus, when implanted in the target tissue, the medical device of the present invention is capable of preventing coagulation or thrombus formation.

Even when not in contact with the blood, the first coating layer is expected to provide a biocompatible surface, reducing unwanted effects associated with the implantation of the implant (which represents a foreign surface) as demonstrated by Lappegard, K. T 2008, J. Biomed. Mater. Res. Vol 87, 129-135. Thus, following implantation and elution of the paclitaxel (and organic additive i.e. dissipation of the second particulate coating layer) the surface that remains is expected to be biocompatible e.g. as demonstrated using Test Method O.

Thus, as illustrated in Examples 14 and 19, an implantable medical device of the present invention exhibits dual activity - the elutable paclitaxel in the second particulate coating layer provides a therapeutic effect, typically an anti-restenosis effect, while the first coating layer comprising immobilized heparin exhibits an antithrombotic effect. The first coating layer further provides the implantable medical device with a biocompatible surface.

As shown in Example 8, the implantable medical device of the present invention is stable to sterilization. In particular, the paclitaxel, when formulated in the second particulate coating layer survives a sterilization process essentially intact.

Suitable sterilization processes include, but are not limited to sterilization using ethylene oxide, vapour hydrogen peroxide, plasma phase hydrogen peroxide, dry heat, autoclave steam sterilization, chlorine dioxide sterilization, gamma ray sterilization or electron beam sterilization. In one embodiment, the paclitaxel is essentially intact after ethylene oxide sterilization, vapour hydrogen peroxide sterilization, plasma phase hydrogen peroxide sterilization or electron beam sterilization. In one embodiment, the therapeutic agent stable to ethylene oxide sterilization, vapour hydrogen peroxide sterilization, plasma phase hydrogen peroxide sterilization or electron beam sterilization (or indeed multiple sterilization methods). The method of sterilization will typically be selected based on the composition of the medical device material and the coating components e.g. ePFTE will be degraded by gamma radiation. Sterilization using ethylene oxide is the most commonly utilized, proven and readily available sterilization technique for implantable medical devices such as stents and stent grafts. Thus, in one embodiment, the paclitaxel is essentially intact after sterilization using ethylene oxide. In another embodiment, the paclitaxel is stable to ethylene oxide sterilization.

Paclitaxel is defined as being essentially intact after sterilization, or is considered to be stable to sterilization, if it exhibits no more than 20% degradation after sterilization without aging, for example no more than 15%, 10%, 9%, 8%, 7%, 6%, 5%, 4%, 3%, 2% or 1% degradation (by weight). Paclitaxel is considered to be degraded if it is chemically altered following sterilization. Conversely, paclitaxel in the second particulate coating layer is defined as being essentially intact after sterilization, or is considered to be stable to sterilization, if the coating retains at least 80% of the paclitaxel chemical content after sterilization, for example at least 85%, 90%, 91%, 92%, 93%, 94%, 95%, 96%, 97%, 98%, 99% or substantially all of the paclitaxel chemical content (i.e. weight) after sterilization.

The amount of intact paclitaxel in the coating following sterilization can be determined using high-performance liquid chromatography (HPLC) techniques such as ultra-performance liquid chromatography (UPLC), for example using the UPLC method described in the Evaluation methods section, and/or by mass spectrometry. Specific methods of quantifying paclitaxel in a coating are provided in Test Methods C-I and C-II.

Specific evaluation methods "Test Method D", "Test Method E", "Test Method F", and "Test Method G" are provided in the Test Methods section for assessing stability to sterilization using ethylene oxide, electron beam, vapour hydrogen peroxide, and plasma hydrogen peroxide, respectively.

In one embodiment, at least 80%, such as at least 85%, 90% or 95% of the paclitaxel chemical content (determined using Test Method C-II) is retained following sterilization using Test Method D.

In one aspect of the invention is provided an implantable medical device as described herein which has been sterilized, e.g. ethylene oxide sterilized.

As shown in Example 8, following sterilization using ethylene oxide according to Test Method D, essentially no degradation of paclitaxel was observed for a stent-graft of the invention coated according to Examples 3a, 3b and 3c. When stent-grafts were subjected to manipulation followed by sterilization, any loss of paclitaxel activity was attributed to the manipulation step, as described in Example 9. Examples 10 and 11 comparing paclitaxel release profiles of stent-grafts of the invention also show that the stent-grafts are unaffected by sterilization by ethylene oxide.

### Therapeutic methods

Implantable medical devices as described hereinabove are of use in medical therapy.

In one aspect of the invention is provided an implantable medical device as described hereinabove for use in treating tissue in the human or animal body. The tissue to be treated includes any body cavity, space, or hollow organ passage(s) such as blood vessels, the urinary tract, the intestinal tract, nasal cavity, neural sheath, intervertebral regions, bone cavities, esophagus, intrauterine spaces, pancreatic and bile ducts, rectum, and those previously intervened body spaces that have implanted vascular grafts, stents, prosthesis, or other type of medical implants.

The implantable medical device as described herein can be of use in the removal of obstructions such as emboli and thrombi from blood vessels, as a dilation device to restore patency to an occluded body passage, as an occlusion device to selectively deliver a means to obstruct or fill a passage or space, and as a centering mechanism for transluminal instruments like catheters.

In one aspect of the invention is provided an implantable medical device as described hereinabove for use in the prevention or treatment of stenosis or restenosis in a blood vessel of the human body. In another aspect of the invention is provided an implantable medical device as described hereinabove for use in the prevention or treatment of stenosis or restenosis in a blood vessel of the human body, where previously placed eluting constructs have failed. In another embodiment, an implantable medical device as described hereinabove can be used to establish or maintain arteriovenous access sites, e.g., those used during kidney dialysis. In a further embodiment, a vascular graft or a stent-graft may be used to redirect flow around an area of blockage or vessel narrowing. In another embodiment, a stent-graft may be deployed to restore patency to an area of diseased vessel or to exclude an aneurysm. In yet another embodiment, a stent device may be deployed to reinforce a diseased vessel following angioplasty.

In one embodiment, said implantable medical device as described hereinabove can be used for Percutaneous Transluminal Angioplasty (PTA) in patients with obstructive disease of the peripheral arteries.

Disclosed is a method for the prevention or treatment of stenosis or restenosis which comprises implanting into said blood vessel in the human body a medical device as described hereinabove.

The second particulate coating layer of the implantable medical device comprises a single loading of paclitaxel. The dose of paclitaxel delivered will depend on many factors including the size of the coated area, the length of time the device is in contact with the target tissue and the amount of paclitaxel in the coating. Suitably the medical device has a second particulate coating layer containing an average of 0.1-10 µg/mm² of paclitaxel, such as 0.2-8 µg/mm², 0.5-5 µg/mm², or 1-4 µg/mm² e.g. 0.5 µg/mm², 1 µg/mm², 2 µg/mm², 3 µg/mm² or 4 µg/mm² of paclitaxel. The apparent coated surface area does not take account of porosity considerations of a porous substrate material. If the substrate material is porous, the effect of porosity on surface area is not considered for these calculations. For example, the apparent surface area of a cylindrical tubular ePTFE vascular graft (which is made of a porous material) with second particulate coating layer comprising the inner surface of the tubular graft is calculated as it is for any cylindrical geometry as 2πrl: where r is the graft inner radius; L is the axial length; and π is the number pi. It is important to note that the porous nature of ePTFE and similar porous materials such as knitted, woven or braided polyester, and their effect on surface area is not accounted for herein. Accordingly, both porous and non-porous substrate materials that are cut into squares for analysis are taken to have a surface area of the length multiplied by the width.

The implantable medical device of the invention will typically contain 0.025-300 mg of paclitaxel in total, for example 0.025-250 mg, 0.05-200 mg, 0.05-150 mg, 0.05-100 mg, 0.1-90 mg, 0.1-80 mg, 0.1-70 mg, 0.1-60 mg, 0.1-50 mg, 0.1-40 mg, 0.1-30 mg, 0.2-20 mg, 0.2-10 mg or 0.2-5 mg e.g. 0.1-300 mg of paclitaxel in total, for example 0.1-250 mg, 0.1-200 mg, 0.1-150 mg, 0.1-100 mg, 0.1-90 mg, 0.1-80 mg, 0.1-70 mg, 0.1-60 mg, 0.1-50 mg, 0.1-40 mg, 0.1-30 mg, 0.2-20 mg, 0.2-10 mg or 0.2-5 mg.

In one embodiment, the coated medical device is a stent and the coating layer contains 10 mg of paclitaxel in total. In one embodiment, the coated medical device is a stent graft and the coating layer contains 10 mg of paclitaxel in total. In one embodiment, the coated medical device is a stent-graft and the coating layer contains 25 mg of paclitaxel in total.

In one embodiment, at least 80% (by weight) of the paclitaxel initially loaded on the medical device of the invention elutes from the device, 28 days after implantation. In another embodiment, at least 50 % (by weight) of the paclitaxel initially loaded on the medical device of the invention elutes from the device, 24 hours after implantation.

### Further embodiments of the invention

An implantable medical device with a surface having a coating comprising:
a first coating layer comprising an immobilized heparin moiety and
a second particulate coating layer comprising elutable paclitaxel and at least one organic additive,
wherein the first coating layer is applied to a portion of the luminal side and to a portion of the abluminal side of the medical device; and
wherein at least a portion of the second particulate coating layer is in contact with at least a portion of the first coating layer.

A stent or stent-graft having a coating comprising:
a first coating layer comprising an immobilized heparin moiety; and
a second particulate coating layer comprising elutable paclitaxel and at least one organic additive;
wherein, the first coating layer is applied to a portion of the luminal side and to a portion of the abluminal side of the stent or stent-graft; and the second particulate coating layer is applied only to a portion of the abluminal side of the stent or stent-graft, and
wherein at least a portion of the second particulate coating layer is in contact with at least a portion of the first coating layer.

A stent or stent-graft having a coating comprising:
a first coating layer comprising an immobilized heparin moiety; and
a second particulate coating layer comprising elutable paclitaxel and at least one organic additive;
wherein at least a portion of the second particulate coating layer is in contact with at least a portion of the first coating layer; and
wherein the organic additive is independently selected from the list consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, adipic acid, glutaric acid, theophylline, and saccharin sodium.

A stent or stent-graft having a coating comprising:
a first coating layer comprising an immobilized heparin moiety; and
a second particulate coating layer comprising elutable paclitaxel and at least one organic additive selected from caffeine and succinic acid;
wherein at least a portion of the second particulate coating layer is in contact with at least a portion of the first coating layer.

An implantable medical device having a coating comprising:
a first coating layer comprising an immobilized heparin moiety; and
a second particulate coating layer comprising elutable paclitaxel and at least one organic additive selected from caffeine and succinic acid, wherein the second particulate coating layer is applied on top of at least a portion of the first coating layer.

A stent or stent-graft having a coating comprising:
a first coating layer comprising an immobilized heparin moiety; and
a second particulate coating layer comprising elutable paclitaxel and at least one organic additive selected from caffeine and succinic acid, wherein the second particulate coating layer is applied on top of at least a portion of the first coating layer.

A stent or stent-graft having a coating comprising:
a first coating layer comprising an immobilized heparin moiety; and
a second particulate coating layer comprising elutable paclitaxel and at least one organic additive;
wherein, the first coating layer is applied to the entire luminal side and to the entire abluminal side of the stent or stent-graft; and the second particulate coating layer is applied only to a portion of the abluminal side of the stent or stent-graft; and
wherein at least a portion of the second particulate coating layer is in contact with at least a portion of the first coating layer.

A process for preparing a coated implantable medical device comprising the steps of:
i)
   a) treating the medical device to provide a polymer coating layer; and then
   b) reacting said polymer layer with a heparin moiety to immobilize the heparin moiety to the polymer coating layer; and further
ii)
   A) dissolving the paclitaxel and the at least one organic additive in a solvent to form a solution; and then
   B) applying the solution to the implantable medical device; and then
   C) evaporating the solvent.
wherein at least a portion of the second particulate coating layer is in contact with at least a portion of the first coating layer.

A process for preparing a coated implantable medical device comprising the steps of:
i)
   a) treating the medical device to provide a polymer coating layer; and then
   b) reacting said polymer layer with a heparin moiety to immobilize the heparin moiety to the polymer coating layer; and further
ii)
   A) dissolving the paclitaxel and the at least one organic additive in a solvent to form a solution; and then
   B) applying the solution to at least a portion of the implantable medical device covered in step (i); and then
   C) evaporating the solvent.

### Advantages

Implanted medical devices according to the present invention at least in some embodiments are expected to have one or more of the following merits or advantages:
- good coating adherence (in particular the second particulate coating layer) such that loss of paclitaxel following compaction and constraining e.g. as measured using Test Method H-I or Test Method I-I is minimal, thereby allowing a lower paclitaxel dosage to be utilized;
- good coating adherence (in particular the second particulate coating layer) such that loss of paclitaxel is minimal during deployment, e.g. as measured using Test Method H-II or Test Method I-II, thereby allowing a lower paclitaxel dosage to be utilized;
- good coating durability (as indicated by good coating adherence);
- the second particulate coating layer is expected to exhibit improved adhesion when coating onto a layer of immobilized heparin moiety (first coating layer) as compared with a second particulate coating layer coated on a coating layer not containing immobilized heparin moiety, e.g. a coating layer of just polymer, such as a coating layer of polyamine, or a device with no coating layer, or a device which has just been washed;
- the first coating layer comprising an immobilized heparin moiety acts as a "wetted" or "wetted-out" surface (i.e. provides a surface with having a lower resistance to fluid transfer), which, when the second particulate coating layer is applied on top of the first coating layer comprising an immobilized heparin moiety, provides a resulting coating layer which is smooth and even;
- a smooth and even coated surface, even following manipulation (e.g. as determined using SEM visualisation techniques);
- suitable paclitaxel release characteristics, in particular even paclitaxel-to-tissue transfer and distribution upon contact with the target tissue e.g. as measured in Test Method A;
- sustained release of the paclitaxel into the target tissue once the medical device has been implanted;
- good coating stability to sterilization (in particular the second particulate coating layer, more particularly the paclitaxel therein) e.g. as measured using Test Method D (ethylene oxide sterilization), Test Method E (electron beam sterilization), Test Method F (vapour hydrogen peroxide sterilization) or Test Method G (plasma hydrogen peroxide sterilization);
- good antithrombotic activity following implantation, which is maintained following release of the second particulate coating layer; (e.g. following Test Method J or Test Method K);
- eventual complete degradation and/or dissipation of the second particulate coating layer following implantation;
- relatively low initial loadings (doses) of paclitaxel on the implantable medical device;
- good biocompatibility, even following degradation and/or dissipation of the second particulate coating layer e.g. as evidenced by the ability to reduce inflammation (e.g. following Test Method O).

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

### Definitions and Abbreviations

- DSC: differential scanning calorimetry
- ePTFE: expanded polytetrafluoroethylene
- h: hour
- HPLC: high-performance liquid chromatography
- ND: not determined
- N/T: not tested
- PABA: p-aminobenzoic acid
- PEG: polyethylene glycol
- PBS: phosphate buffered saline
- PLGA: poly(lactic-co-glycolic) acid
- PVP: polyvinylpyrrolidone
- SEM: scanning electron microscopy
- UPLC: ultra-performance liquid chromatography
- PTX: paclitaxel

### EXAMPLES

### GENERAL PROCEDURES

### Chemicals

Anhydrous crystalline paclitaxel was purchased from Indena. Paclitaxel dihydrate may be purchased from Sigma-Aldrich. Patent Blue V was purchased from Fluka. Anydrous Caffeine USP 98.5 - 101.0 % was purchased from Spectrum chemicals MFG. CORP. Succinic acid ACS Reagent, ≥990 % was purchased from Sigma-Aldrich.

### Solvent

Acetone ("dry" with <0.5% water) was purchased from Sigma. Water was deionized before use.

### Materials

Stent-grafts with an immobilized heparin coating with dimensions of 5 mm in diameter and 25 mm in length, and 6 mm diameter and 25 mm in length were obtained from W.L. Gore and Associates Inc. Stent-grafts without an immobilized heparin coating with dimensions of 5 mm in diameter and 25 mm in length were obtained from W.L. Gore and Associates Inc. Porcine carotid arteries were obtained from Lövsta Kött AB (Uppsala, Sweden).

### Evaluation methods

The parameter being evaluated by each method is given in parentheses.

### Differential Scanning Calorimetry (DSC) analysis (peak melting endotherm determination)

A solid sample is added to a DSC pan. The mass of the sample is weighed, and the pan sealed with pinhole lids. The sample is examined using DSC (model #Q2000, TA Instruments), by equilibrating at 25°C, ramping 10°C/min to 100°C, dwelling at 100°C for 20 min (to remove any trace solvent, in particular acetone or acetone:water), ramping 10°C/min to 225°C.

### Ultra-Performance Liquid Chromatography (UPLC) analysis (paclitaxel concentration)

UPLC analysis is carried out using a Waters instrument (model #ACQUITY H-class). The identification of paclitaxel is determined by the retention time of paclitaxel. The concentration of paclitaxel is directly proportional to the integrated peak area, which was determined by external standardization. Samples are dissolved in a sample diluent or submerged in an extraction solvent and sonicated for 15 minutes. Paclitaxel standards are prepared by serial dilution of pure paclitaxel dissolved in the sample diluent. All samples and standards are protected from light during preparation. UPLC chromatography parameters are: phenyl column (1.7 um, 2.1 x 50mm); mobile phase water:acetonitrile; flow rate 0.7 ml/min; run time 12 min; injection volume 3 ul; purge solvent acetonitrile:water (5:95 v/v); wash solvent isopropanol; column temperature 35°C; UV detector wavelength 227.0 ±1.2 nm; sample rate 20 points/sec.

### Scanning electron microscopy with energy dispersive X-ray spectroscopy (Coating coverage and uniformity)

SEM images of coated devices of the invention are captured using a Hitachi TM3000 table top SEM.

### X-ray photoelectron spectroscopy with depth profiling (XPS) (coating thickness)

X-ray Photoelectron Spectroscopy (XPS or ESCA) is the most widely used surface characterization technique providing non-destructive chemical analysis of solid materials. Samples are irradiated with mono-energetic X-rays causing photoelectrons to be emitted from the top 1 - 10nm of the sample surface. An electron energy analyzer determines the binding energy of the photoelectrons. Qualitative and quantitative analysis of all elements except hydrogen and helium is possible, at detection limits of ~ 0.1 - 0.2 atomic percent. Analysis spot sizes range from 10µm to 1.4mm. It is also possible to generate surface images of features using elemental and chemical state mapping. Depth profiling is possible using angle-dependent measurements to obtain non-destructive analyses within the top 10nm of a surface, or throughout the coating depth using destructive analysis such as ion etching.

### Test methods

### Test Method A - In vitro tissue transfer and uptake test of paclitaxel

Coated implantable medical devices, such as stents or stent-grafts are examined for their ability to transfer paclitaxel from the stent-graft surface to a vascular tissue in an *in vitro* model as essentially described by Liao (D. Liao et al., Biochem Biophys Res Commun, 372(4): 668-673, 2008. "Vascular smooth cell proliferation in perfusion culture of porcine carotid arteries"). A procedure which may be used when the implantable medical device is a stent or stent-graft is as follows. The coated stent or stent-graft is compacted diametrically according to Test Method H-I or I-I, respectively. The compacted stents or stent-grafts are inserted into the proximal end of the porcine vessel to the middle of the vessel, and deployed to their expanded state according to Test Method H-II or I-II, respectively. A Luer fitting is attached to the proximal end and distal ends of the vessel with wax thread. Tubing was connected to the proximal and distal fittings, and the vessel is flushed with PBS at 60 ml/min for 24 hr at 37 °C. The stent or stent-graft is removed, and vessel was analyzed for paclitaxel content using the UPLC technique described in Evaluation Methods.

### Test Method B - Blood contact evaluation (platelet loss)

Blood contact evaluation is performed on an implantable device of the invention, in particular on the implantable device coated with both the first and the second layer, to evaluate its thromboresistant properties. A procedure which may be used when the implantable medical device is a stent-graft is as follows. Firstly the stent-graft is washed with 0.15M saline solution for 15 min to ensure complete wetting. The wetted stent-graft is placed in heparinized PVC tubing containing whole blood and left to rotate in a circulating loop at 20 rpm (see Ekdahl K. N., Advances in Experimental Medicine and Biology, 2013, 735, 257-270 for a representative procedure). The platelets from fresh blood and from the blood collected from the tubes are counted in a cell counter to measure the loss of platelets. A great loss of platelets indicates poor thromboresistant performance of the device, in particular of the first coating layer. Conversely a minimal loss of platelets indicates a thromboresistant device, in particular with an thromboresistant first coating layer.

The negative control is an empty loop of heparinized PVC without any device. This represents a thromboresistant control for which the incubated blood should only demonstrate a minimal loss of platelets. The positive control is an empty loop of non-heparinized PVC without any device. This represents a thrombogenic control for which a great loss of platelets should be observed. The controls are included for ensuring the quality of the experiment and the blood.

### Test Methods C - Determining paclitaxel content of second particulate coating layer following manipulation

These tests allow the amount of paclitaxel on the coated device (in particular in the second particulate coating layer) to be determined. By comparing the amount of paclitaxel on the device before and after device manipulation, the durability of the coating, in particular of the second particulate coating layer, can be assessed.

### Test Method C-I - weight

The coated device is weighed before and after manipulation (e.g. manipulation according to Test Methods H or I). The weight of the second particulate coating layer lost during manipulation can therefore be determined. In cases where the coating composition prior to manipulation is known (e.g. wherein the second particulate coating layer consists of paclitaxel and a single organic additive, and the amount (and molecular weight) of each present in the coating is known), then the weight of paclitaxel lost can calculated, as can the % of paclitaxel lost, and the % of paclitaxel remaining on the device.

### Test method C-II - extraction

The device is manipulated (e.g. according to Test Methods H or I) and then the paclitaxel remaining on the device (in the second particulate coating layer) following manipulation is extracted by immersing the device in acidified methanol (0.2 v% acetic acid in 5 mL methanol) for 15 minutes. The paclitaxel-containing methanol solution is evaluated using UPLC techniques (as described in Evaluation Methods) to determine the paclitaxel content. This can be compared with the known loading of paclitaxel on the device prior to manipulation, and the % paclitaxel lost, and the % of paclitaxel remaining on the device may be calculated. This method may also be used (without the manipulation step) to determine if the paclitaxel in the second particulate coating layer is considered to be "elutable".

### Test Method D - Stability to ethylene oxide

The implanted medical device of the invention is placed in a breathable polyethylene pouch (e.g. a Tyvek pouch) and subjected to at least 12 hours preconditioning at 50 °C and 60% relative humidity followed by 2 hours exposure of ethylene oxide at a pressure of 366 mBar and 50 °C. The chamber is then degassed at 50 °C for at least 10 hours. Sterilization by ethylene oxide may be performed at Synergy Health Ireland Ltd.

After sterilization, the paclitaxel content on the device is assessed (through device extraction i.e. immersion of the whole device in an extraction solvent) using UPLC quantification as described in the evaluation methods section. For each device, the percentage paclitaxel recovery after sterilization is calculated by normalizing the extracted paclitaxel amount by the theoretical paclitaxel amount loaded on the device pre-sterilization.

### Test Method E - Stability to electron beam sterilization

A further method to sterilize an implantable medical device of the invention is electron beam sterilization. The device is placed into a breathable polyethylene pouch (e.g. a Tyvek pouch) and irradiated at a dosage of 15 to 40 kGray under ambient conditions, using commercial sterilization providers, such as Sterigenics International, Inc. (Deerfield, Illinois). After e-beam sterilization, the paclitaxel content on the device is assessed as described for Test Method C-II.

### Test Method F - Stability to vapour hydrogen peroxide sterilization

A further method to sterilize an implantable medical device of the invention is vapour hydrogen peroxide sterilization. The device is placed into a breathable polyethylene pouch (e.g. a Tyvek pouch) and exposed to vapour hydrogen peroxide using a commercially available sterilization chamber, such as the VHP-MD880 system (Steris Corp., Mentor, Ohio) following the manufacturer's recommended protocol. After vapour hydrogen peroxide sterilization, the paclitaxel content on the device is assessed as described for Test Method C-II.

### Test Method G - Stability to plasma hydrogen peroxide sterilization

A further method to sterilize an implantable medical device of the invention is plasma phase hydrogen peroxide sterilization. The implantable medical device is placed into a breathable polyethylene pouch (e.g. a Tyvek pouch) and exposed to plasma phase hydrogen peroxide using a commercially available sterilization chamber, such as the Sterrad 100NX system (Advanced Sterilization Products, Irvine, California) following the manufacturer's recommended protocol. After plasma phase hydrogen peroxide sterilization, the paclitaxel content on the device is assessed as described for Test Method C-II.

### Test Methods H-I, H-II, I-I and I-II manipulation of stents and stent-grafts

The impact of manipulation on a stent or stent-graft (e.g. during typical manufacturing processing and then implantation) can be assessed by comparing, for example, the weight of the entire stent or stent-graft before and after manipulation, or the amount of paclitaxel on the stent or stent-graft before and after manipulation (using Test Method C-I or C-II). Stents are manipulated according to Test Method H-I, or Test Method H-I followed by Test Method H-II, and stent-grafts are manipulated according to Test Method I-I, or Test Method I-I followed by Test Method I-II.

### Test Method H-I - Compaction and constraining of stents

Stents are compacted diametrically to an outer diameter of 3.36 mm using means known to those of skill in the art of self-expanding stents and stent-grafts. Once compacted according to Test Method H-II, the stents are constrained in the compacted state within a constraint tube with an inner diameter of 3.6 mm.

### Test Method H-II - Deployment of stents

Stents are deployed from the containment tube with the use of a push rod. When the stent is pushed out from the containment tube, the stent shears against the containment tube inner surface

### Test Method I-I - Compaction and constraining of stent-grafts

Stent-grafts are compacted diametrically to an outer diameter of 3.0 mm using means known to those of skill in the art of self-expanding stent-grafts. Once compacted according to Test Method I-I, stent-grafts are constrained in the compacted state within a constraint tube with an inner diameter of 3.0 mm.

### Test Method I-II - Deployment of stent-grafts

Stent-grafts are deployed by pulling them out using attached wax threads. When the stent is pulled out of the constraint tube, the stent shears against the sharp edges at the outlet.

### Test Method J - Evaluation of heparin moiety bioactivity via HCII binding activity (quantitative heparin function)

The heparin bioactivity of a device, in particular the first coating layer comprising an immobilized heparin moiety can be measured according to WO2009/064372 (Gore Enterprise Holdings, Inc.) by measuring the ability, or capacity, of the heparin moiety to bind a known quantity of heparin cofactor II (HCII), using an assay as described by Larsen M.L., et al., in "Assay of plasma heparin using thrombin and the chromogenic substrate H-D-Phe-Pip-Arg-pNA (S- 2238)." Thromb Res 13:285-288 (1978) and Pasche B., et al., in "A binding of antithrombin to immobilized heparin under varying flow conditions." Artif. Organs 1991; 15:281 -491). The results are expressed as picomoles heparin cofactor II (HCII) bound per apparent square centimetre of device surface (pmol HCII/cm² device surface). The apparent device surface area does not take into account multiple covered surfaces nor porosity considerations of a device composed of a porous material. If the surface of the device is porous, the effect of porosity on surface area is not considered for these calculations. For example, the apparent surface area of a cylindrical tubular ePTFE vascular graft (which is made of a porous material) with heparin immobilized on substrate material comprising the inner surface of the tubular graft is calculated as it is for any cylindrical geometry as 2πrL: where r is the graft inner radius; L is the axial length; and π is the number pi.

### Test Method K - Evaluation of heparin moiety bioactivity via ATIII binding activity (quantitative heparin function)

The heparin bioactivity of a device, in particular the first coating layer comprising an immobilized heparin moiety is measured as the capacity of the surface bound heparin to bind antithrombin III (ATIII) as described by Pasche, et al. in "A binding of antithrombin to immobilized heparin under varying flow conditions" (Artif. Organs 1991; 15:281 -491) and Larsen M. L., et al. in "Assay of plasma heparin using thrombin and the chromogenic substrate H-D-Phe-Pip-Arg-pNA" (S-2238) (Thromb. Res. 1978; 13:285-288). Washed samples are incubated with an excess antithrombin in solution to saturate all available antithrombin-binding sites of the heparin surface. Non-specifically adsorbed antithrombin is rinsed away using a salt solution. Subsequently, antithrombin specifically bound to the surface bound heparin is released by incubating with a solution of heparin at high concentration. Finally, the antithrombin released from the heparin-surface is measured in a thrombin inhibition assay, based on a chromogenic thrombin substrate. The results are expressed as picomoles antithrombin III (ATIII) bound per apparent square centimetre of device (pmol ATIII/cm² device surface). The apparent device surface area does not take into account multiple covered surfaces nor porosity considerations of a device composed of a porous material. If the surface of the device is porous, the effect of porosity on surface area is not considered for these calculations. For example, the apparent surface area of a cylindrical tubular ePTFE vascular graft (which is made of a porous material) with heparin immobilized on substrate material comprising the inner surface of the tubular graft is calculated as it is for any cylindrical geometry as 2πrL: where r is the graft inner radius; L is the axial length; and π is the number pi.

### Test Method L - Evaluation of heparin moiety density (quantitative heparin attachment)

Quantification of surface immobilized heparin can be performed by complete degradation of heparin followed by colorimetric determination of the reaction products released into solution. Degradation is achieved by reacting the heparin surface with an excess of sodium nitrite under acidic conditions. The degradation products, mainly disaccharides, are quantified colorimetrically in a reaction with MBTH (3-metyl-2-bezotiazolinon hydrazone hydrochloride), essentially as described in Smith R.L. and Gilkerson E (1979), Anal Biochem 98, 478-480.

### Test Method M - In vitro evaluation of paclitaxel elution profile

In order to study the release rate of paclitaxel from the device (in particular from the second particulate coating layer) *in vitro,* the profile of accelerated elution can be studied. For this purpose, the coated device is put in a solution of a suitable buffer at a fixed temperature. The eluted paclitaxel is dissolved in the aqueous buffer solution containing cyclodextrine which increases the solubility of paclitaxel in water up to the necessary concentration. Withdrawal of samples at chosen time points, analysis of the paclitaxel content by UPLC techniques (as described in Evaluation Methods and Test Method C-II) for paclitaxel content and plotting of paclitaxel content against time, an elution profile is created.

### Test Method N - Staining techniques

Devices of the invention can be subjected to toluidine blue stain solution (200 mg/L in water) by immersing in the solution for 2 minutes followed by extensive water rinse. A blue or violet colour is observed on surfaces that contain a net negative charge e.g. immobilized heparin moiety.

### Test Method O - Surface biocompatibility

The biocompatibility of a surface of an implantable medical device of the invention with a first coating layer and second particulate coating layer can be assessed as described in Lappegard, K. T 2008, J. Biomed. Mater. Res. Vol 87, 129-135. A procedure which may be used to evaluate the inflammatory response of a stent-graft of the invention following removal of the second particulate coating layer (according to Test Method C-II) is as follows. Firstly the stent-graft is washed with 0.15 M saline solution for 15 min. The wetted stent-graft is placed in heparinized PVC tubing containing whole blood and left to rotate in a circulating loop at 20 rpm (see Ekdahl K. N., Advances in Experimental Medicine and Biology, 2013, 735, 257-270 for a representative procedure). After incubation, the blood is centrifuged for 15 min, 3220 g at 4°C. The plasma is frozen in aliquots at -70°C for later analysis of cytokines. Plasma samples are analyzed using multiplex cytokine assay (Bio-Plex Human Cytokine 27-Plex Panel, Bio-Rad Laboratories, Hercules, CA) according to the method described by Lappegard et al. (above).

The negative control is an empty loop of heparinized PVC without any device. This represents a non-inflammatory control for which the incubated blood should demonstrate no or minimal amount of inflammatory markers. The positive control is an empty loop of non-heparinized PVC without any device. This represents an inflammatory control for which a greater amount of inflammatory markers should be observed. The controls are included for ensuring the quality of the experiment and the blood.

### Example 1: Methods for preparing immobilized heparin coating (first coating layer) on an implantable medical device

The surface of the medical device to be coated is cleaned with isopropanol and an oxidizing agent. The surface is then treated using the method described in Larm et al. in EP-B-0086186 and EP-B-495820 to form coating bilayers ending with a layer of sulfated polysaccharide.

The bilayers are built-up by alternating adsorption of a positively charged polyamine (polyethyleneimine (e.g. as used in the examples of EP0495820B1) and negatively charged sulfated polysaccharide (dextran sulfate). Polyethyleneimine is diluted with water to prepare a stock solution (5 g polyethyleneimine was added to 20 mL purified water). The polyamine is cross-linked with a di-functional aldehyde (crotonaldehyde). Every pair of polyamine and sulfated polysaccharide is called one bilayer. The surface of the device is primed with four bilayers, the final layer being dependent on the subsequent method of immobilizing the heparin moiety.

### Immobilization of heparin as described in EP-B-0086186 - via reductive amination. Heparin is subjected to degradation by diazotation to form terminal (end point) free aldehyde group, which subsequently reacts via the aldehyde with an amino group on the surface of the implantable medical device to form a Schiff base which is converted to a secondary amine linker by reduction.

A solution of heparin (1 g) in 300 ml water is cooled to 0°C on an ice bath. Sodium nitrite (10 mg) is added with stirring. Then acetic acid is added drop-wise (2 ml). The solution is allowed to stand under stirring for two more hours at 0°C. The reaction mixture is worked up by dialysis against distilled water and lyophilization to produce end-point aldehyde-functionalized heparin.

The surface of the device to be heparinized is primed with four bilayers as described above, ending with a final layer of polyethyleneimine (e.g. as used in the examples of EP0495820B1). Following rinsing, the surface to be coated is incubated with a solution of the end-point aldehyde-functionalized heparin (2-20 mg/mL) and sodium cyanoborohydride (0.5 mg/ml) in a phosphate buffer at pH 7.0 for 24 hours at room temperature. The heparinized surface is carefully rinsed with water.

### Immobilization as described in WO2011/110684 - via a thioether linker. Thiol-functionalized heparin is reacted with a maleimide-functionalized polyamine surface

Thiol-functionalized heparin is prepared as follows. Nitrite-degraded heparin with end-point aldehyde groups (prepared as described above) (5.00 g, 1.0 mmol), cysteamine hydrochloride (0.57 g, 5.0 mmol) and sodium chloride (0.6 g) are dissolved in purified water. The pH is adjusted to 6.0 with 1 M NaOH (aq) and 1 M HCl (aq). To the solution is added 3.1 ml of 5 % (aq.) NaCNBH₃ (0.16 g, 2.5 mmol) and the reaction is stirred overnight at room temperature. The pH is adjusted to 11.0 with 1 M NaOH (aq) and the resulting product is dialyzed against purified water with a SpectraPor dialysis membrane mwco 1kD (flat width 45mm) for three days. The reaction mixture is then concentrated and freeze dried to obtain 2.6 g of the thiol-functionalized heparin (at the C1 of the reducing terminal) as a white fluffy powder.

Maleimide-functionalized polyethyleneimine (polyethyleneimine as used in the examples of EP0495820B1 (above)) is prepared as follows. 4-maleimidobutyric acid (0.50 g, 2.7 mmol) and N-hydroxysuccinimide (NHS) (0.32 g, 2.7 mmol) are dissolved in 3 mL of dichloromethane and stirred at 0°C. A solution of N,N'-dicyclohexylcarbodiimide (0.56 g, 2.7 mmol) in 3 mL of dichloromethane is added slowly to the reaction mixture at 0°C. The reaction mixture is stirred overnight and the byproducts are filtered off and the NHS activated 4-maleimidobutyric acid is concentrated and dried under vacuum. The dried NHS activated 4-maleimidobutyric acid is dissolved in 30 mL of purified water and mixed with 7.6 mL of the polyethyleneimine stock solution at 0°C and left to react overnight at room temperature to obtain a 1 % solution of the maleimide functionalized polyethyleneimine.

The surface of the device to be heparinized is primed with four bilayers as described above, ending with a final layer of negatively charged sulfated polysaccharide (dextran sulfate). Then next coating step uses a solution of 10 mL of a 1 % solution of the maleimide-functionalized polyethyleneimine in 1000 mL of a 0.04 M/0.04 M borate/phosphate buffer at pH 8.0. The adsorption of the maleimide-functionalized polyethyleneimine to the sulfate surface is carried out for 20 minutes at room temperature. A two minute water rinse is performed after the adsorption to rinse off excess polymer. 500 mg of thiol functionalized heparin is dissolved in 1000 mL of de-ionized water and 50 mg tris(2-carboxyethyl)phosphine hydrochloride, 500 mg 4,4'-azobis(4-cyanovaleric acid), and 2.9 g NaCl were added. The pH is adjusted to 3.7 with 1 M HCl (aq).

The reaction between the solution of the thiol-functionalized heparin and the maleimide functionalized polyethyleneimine surface is carried out at 70°C for 3h. Purification is performed by rinsing off non-covalently linked heparin for 10 minutes using a 0.04 M/0.04 M borate/phosphate buffer at pH 8.0. A final rinse with de-ionized water for two minutes is performed to wash away buffer salt residues. The flow used during the entire process is 100 mL/min.

### Example 2: Method for preparing paclitaxel-organic additive layer (second particulate coating layer) on a stent where the organic additive is caffeine.

Vascular stents (5mm by 30mm), featuring a dual component design, constructed from a single wire nitinol stent interconnected by a durable, biocompatible, expanded polytetrafluoroethylene (ePTFE) structure, were made according to US2009/0182413A1 (Gore Enterprise Holdings, Inc.). The durable, biocompatible, expanded ePTFE structure was coated with a heparin-bonded surface (via reductive amination to form the first coating layer) according to US Patent 6,461,665.

The aforementioned heparin coated vascular stents were over-coated in expanded form with a paclitaxel-excipient coating comprising paclitaxel and caffeine (second particulate coating layer) as follows. Paclitaxel (anhydrate) and caffeine at a weight ratio of 75:25 were dissolved in 90/10 (v/v) acetone/water to obtain a 20 mg/ml paclitaxel solution.

The aforementioned heparin coated vascular stents were tied to a thread at one end for handling during the coating process. They were dipped into the paclitaxel-caffeine solution, removed, and air dried; the coating procedure was repeated 10 to 30 additional times to produce three coated stents. At the end of the coating procedure each coated stent was weighed (stent device 1 had a coating weight of 0.588 mg, stent 2 had a coating weight of 0.642 mg and stent 3 had a coating weight of 1.2 mg) before being examined using DSC. A stent was compacted into a high mass DSC pan and sealed with an o-ring and lid (TA Instruments, part # 900825.902) and analyzed using the DSC method described in General Procedures (except that the sample was not dwelled at 100°C); an uncoated vascular stent was analyzed as a reference. A single depressed melting endotherm at 132°C was observed for the paclitaxel-caffeine solid composition.

### Example 3: Method for preparing paclitaxel-organic additive layer (second particulate coating layer) on a stent-graft

GORE^{®} VIABAHN^{®} endoprostheses with Heparin Bioactive Surface ("Stent-grafts" - as described in "Materials") are stent-grafts which have been pre-coated with a coating layer of immobilized heparin i.e. are purchased pre-coated with the first coating layer of immobilized heparin.

The afore-mentioned pre-coated devices were over-coated in expanded form with paclitaxel-organic additive coatings comprising paclitaxel and succinic acid, or paclitaxel and caffeine, as the second particulate coating layer. Paclitaxel (anhydrate) and the organic additive were co-dissolved in 95/5 (v/v) acetone/water to form a coating solution which was applied to the pre-coated devices as shown in Table 1 (wherein "excipient" refers to the organic additive). Specific coating compositions are described in Examples 3a, 3b and 3c below.

**Table 1 - Coating solution composition**

| **Example** | **Excipient** | **Excipient (wt%)** | **Paclitaxel (wt%)** | **% Paclitaxel* (wt%)** | **Total solids (mg/ml)** |
|---|---|---|---|---|---|
| 3a | Caffeine | 0.04 | 0.12 | 75 | 1.3 |
| 3b | Caffeine | 0.42 | 1.25 | 75 | 13.3 |
| 3c | Succinic acid | 0.12 | 0.37 | 75 | 4.0 |

| | | | | | |
|---|---|---|---|---|---|
| *% of paclitaxel (wt%) in solid components of coating solution | | | | | |

The stent-grafts were coated at the proximal end (covering the portion up to 5 mm from the proximal end) by dispensing the coating solution (25-50 µl of solution, using a syringe pump) under rotation. The final drug loading on the coated area for all devices was approximately 0.32 - 6.37 µg/mm² (estimated by dispensing a known solution volume with a known paclitaxel concentration). Coated stent-grafts can be sterilized according to Test Methods D, F or G.

### Example 3a: Method for preparing paclitaxel-caffeine layer (second particulate coating layer) on a stent-graft with 500 µg paclitaxel loading

100 mg of paclitaxel and 33 mg of caffeine were added to a glass vial. A mixture of acetone (9.5 mL) and water (0.5 mL) was added to form a solution which was allowed to dissolve while stirring at room temperature. The resulting coating solution (10 mg paclitaxel/mL) was applied to the stent-graft using a syringe pump by dispensing 50 µL onto the end of the stent-graft as described in Example 3. The coated stent-graft was thereafter allowed to dry at room temperature overnight.

### Example 3b: Method for preparing paclitaxel-caffeine layer (second particulate coating layer) on a stent-graft with 25 µg paclitaxel loading

100 mg of paclitaxel and 33 mg of caffeine were added to a glass vial. A mixture of acetone (9.5 mL) and water (0.5 mL) were added to form a solution which was allowed to dissolve while stirring at room temperature. The resulting solution was diluted x10 to yield a final coating solution with concentration of 1 mg paclitaxel/mL which was applied to the stent-graft using a syringe pump by dispensing 25 µL onto the end of the stent-graft as described in Example 3. The coated stent-graft was thereafter allowed to dry at room temperature overnight.

### Example 3c: Method for preparing paclitaxel-succinic acid layer (second particulate coating layer) on a stent-graft with 150 µg paclitaxel loading

30 mg of paclitaxel and 10 mg of succinic acid were added to a glass vial. A mixture of acetone (9.5 mL) and water (0.5 mL) were added to form a solution which was allowed to dissolve while stirring the solution under room temperature. The resulting solution (3 mg paclitaxel/mL) was applied to the stent-graft using a syringe pump by dispensing 50 µL onto the end of the stent-graft as described in Example 3. The stent-graft was thereafter allowed to dry at room temperature overnight.

### Example 4: Thermal analysis of second particulate coating layer of implantable medical device

The coated implantable medical devices of the invention can be examined by DSC using the method described in General Procedures. In particular, the thermal behavior of the second particulate coating layer can be analyzed. A sample of the second particulate coating layer can be obtained by scraping the coating with a stainless steel spatula. The particulate sample can then be added to a pre-weighed DSC pan and DSC analysis performed.

In certain embodiments, samples of second particulate coating layer obtained from implantable medical devices of the present invention are expected to exhibit depressed melting endotherms i.e. a depressed melting point which is lower that the melting points of both the paclitaxel and the or each organic additive will be observed.

### Example 5: Adhesion test analysis of second particulate coating layer of stent-graft prepared according to Example 3c

The adhesion of the paclitaxel-succinic acid layer (second particulate coating layer) of the stent-graft prepared according to Example 3c was investigated. Adhesion was assessed by comparing the weight and content of paclitaxel on the stent-graft (according to Test Methods C-I and C-II) before and after compaction and constraining (according to Test Methods I-I and I-II). A lower % of paclitaxel lost (as determined by both Test Methods C-I and C-II) indicates better adhesion and a more durable device, and in particular better adhesion and a more durable second particulate coating layer. To provide comparative references, stent-grafts with no immobilized heparin coating layer (i.e. no first coating layer) or any other coating layer, were also prepared according to Example 3c. The results are summarized in Table 2 and Figure 8.

**Table 2 - Paclitaxel (PTX) content pre- and post- compaction and deployment (test)**

| **Prepared in Example:** | **PTX-excipient pre test** [µg]** | **PTX* pre-test [µg]** | **PTX-excipient post test** [µg]** | **PTX* post test [µg]** | **% PTX loss*** | **% PTX loss**** |
|---|---|---|---|---|---|---|
| Example 3c | 215±1 | 155±3 | 197±5 | 131±5 | 14 | 8 |
| Reference | 182±36 | 150*** | 74±64 | 102±19 | 32 | 59 |

| | | | | | | |
|---|---|---|---|---|---|---|
| * Paclitaxel content determined according to Test Method C-II ** Paclitaxel content determined according to Test Method C-I ***Paclitaxel as loaded on the device based on known concentration and known volume. | | | | | | |

It is evident from Table 2 and Figure 8 that the stent-graft wherein the paclitaxel-succinic acid layer (second particulate coating layer) was applied to a surface of immobilized heparin (first coating layer) has a higher paclitaxel content post compaction, (using both Test Methods C-I and C-II) compared with the stent-graft wherein the paclitaxel-succinic acid layer (second particulate coating layer) was applied to a surface not comprising immobilized heparin.

It is therefore evident that the first coating layer comprising an immobilized heparin moiety enhances the adhesion properties of second particulate coating layer. Without wishing to be bound by theory, the present inventors believe that the first coating layer containing immobilized heparin aids the wetting of the surface which allows for a better contact of the second particulate coating layer to the surface of the medical device.

One skilled in the art would be able to design a coated implantable device with a larger surface area covered by the second coating layer. Similar results in term of adhesion would be expected.

### Example 6: Durability analysis of second particulate coating layer - coated stent

The coated stents of Example 2 underwent compaction and deployment to examine durability and robustness of the coating layer, in particular the second particulate coating layer. The durability was determined by comparing the coating weight before, and after, compaction and deployment.

The coated stents of Example 2 were compacted and constrained according to Test Method H-I and then deployed according to Test Method H-II. After deployment, the stent was weighed and compared to its weight before compaction. The results are shown in Table 3.

**Table 3- Summary of Coating Durability**

| **Stent Device** | **Coating Weight (mg)** | **Coating Lost (mg)** | **% of coating lost** |
|---|---|---|---|
| 1 | 0.588 | 0.085 | 14.5 |
| 2 | 0.642 | 0.038 | 5.9 |
| 3 | 1.2 | 0.107 | 8.9 |

The average coating mass loss was 9.8 % and this was determined to represent a high degree of durability. This durability not only considers the compaction and expansion of the coated stent, but also considers the stent being pushed out from the containment tube, wherein the stent sheared against the containment tube inner surface.

### Example 7: Durability analysis of second particulate coating layer - coated stent-graft

The coated stent-grafts of Examples 3a, 3b and 3c underwent compaction, and were then constrained and deployed, to examine durability and robustness of the coating layer, in particular the second particulate coating layer. The durability was determined by comparing the weight of paclitaxel in the coating before, and after, compaction and deployment (manipulation).

The coated stent-grafts of Examples 3a, 3ba and 3c were compacted and constrained according to Test Method I-I and then deployed according to Test Method I-II. After deployment, the amount of paclitaxel in the coating was determined using Test Method C-II. Results are shown in Table 4.

**Table 4 - Paclitaxel (PTX) content pre- and post-compaction, constraining and deployment (test)**

| **Prepared in example:** | **PTX mean content* pre-test** [µg]** | **PTX mean content* post test** [µg]** | **% PTX loss** |
|---|---|---|---|
| Example 3a | 471 ± 4.6 | 347 ± 17 | 26 ± 4 |
| Example 3b | 23.8 ± 0.7 | 19.1 ± 3.2 | 20 ± 13 |
| Example 3c | 155 ± 2.7 | 129 ± 1 | 17 ± 0.5 |

| | | | |
|---|---|---|---|
| * Paclitaxel content according to Test Method C-II ** Test Method I-I followed by Test Method I-II | | | |

The stent-grafts show good resistance to internal stress indicated by the low amount of paclitaxel that is lost during manipulation. On average, the stent-grafts lost 21% of the paclitaxel in the coating following manipulation, representing a high degree of durability.

### Example 8: Sterilization stability analysis of second particulate coating layer - coated stent-graft

The coated stents of Examples 3a, 3b and 3c were sterilized using ethylene oxide as set out in Test Method D. Following sterilization, the amount of paclitaxel in the coating was determined using UPLC (as described in Test Method C-II) to determine the stability of the implantable medical device, in particular the stability of the second particulate coating layer. The results are shown in Table 5.

**Table 5 - Paclitaxel (PTX) content pre- and post- sterilization**

| **Prepared in example:** | **PTX mean content* pre-sterilization** [µg]** | **PTX mean content* post sterilization** [µg]** | **% PTX loss** |
|---|---|---|---|
| Example 3a | 471 ± 4.6 | 473 ± 0.4 | 0 |
| Example 3b | 23.8 ± 0.7 | 24.8 ± 0.2 | 0 |
| Example 3c | 155 ± 2.7 | 150 ± 0.3 | 3 |

| | | | |
|---|---|---|---|
| * Paclitaxel content according to Test Method C-II ** Sterilized according to Test Method D | | | |

Essentially no degradation of paclitaxel was observed post-sterilization, indicating that the devices of Examples 3a, 3b and 3c devices have excellent paclitaxel stability following ethylene oxide sterilization.

### Example 9: Durability of second particulate coating layer post-sterilization - coated stent graft

The coated stents of Examples 3a, 3b and 3c were sterilized using ethylene oxide as set out in Test Method D. Following sterilization, the stent-grafts were compacted and constrained according to Test Method I-I and then deployed according to Test Method I-II. After deployment, the amount of paclitaxel in the coating was determined using Test Method C-II. The results are shown in Table 6.

**Table 6 - Paclitaxel (PTX) content pre- and post-sterilization and subsequent compaction, constraining and deployment (test)**

| **Prepared in example:** | **PTX mean content* as coated [µg]** | **PTX mean content* post-sterilization** and post-test*** [µg]** | **% PTX loss** |
|---|---|---|---|
| Example 3a | 471 ± 4.6 | 359 ± 32.3 | 24 ± 7 |
| Example 3b | 23.8 ± 0.7 | 22.0 ± 1.6 | 8 ± 7 |
| Example 3c | 155 ± 2.7 | 124 ± 10.6 | 20 ± 7 |

| | | | |
|---|---|---|---|
| * Paclitaxel content according to Test Method C-II ** Sterilized according to Test Method D *** Compaction, constraining and deployment according to Test Methods I-I and I-II | | | |

On average, the coated stents lose 17% of the paclitaxel in the coating following sterilization and compaction, constraining and deployment. Based on the results shown in Table 5 of Example 8, the loss of paclitaxel in the present Example is attributed to the manipulation process rather than the sterilization. The 17% loss of paclitaxel is considered to represent a high degree of durability.

### Example 10: Paclitaxel-organic additive release profile - coated stent graft

The coated stent-grafts of Examples 3b and 3c were immersed in an aqueous acetate buffer containing 30 % cyclodextrin. The release profile of paclitaxel from the device was measured over 24 hours at 37 °C according to Test Method M and the results are shown in Table 7.

**Table 7 - Paclitaxel content in media**

| **Prepared in example:** | **Paclitaxel content in media* [ug]** | | | | | | | | |
|---|---|---|---|---|---|---|---|---|---|
| Timepoint [h] | 0.25 | 0.5 | 1 | 1.5 | 2 | 4 | 6 | 8 | 24 |
| Example 3b | 13 | 15 | 14 | 15 | 18 | 19 | 19 | 19 | 20 |
| Example 3c | 51 | 66 | 73 | 75 | 85 | 94 | 103 | 105 | 122 |

| | | | | | | | | | |
|---|---|---|---|---|---|---|---|---|---|
| Mean of N=2 * Content in media according to Test Methods M and C-II | | | | | | | | | |

The highest rate of paclitaxel release was observed in the initial period up the first time point (0.25 h) with even and sustained (lower) release rates being observed over the remaining period of observation (up to 24 h). An average of 80 % of the PTX load of the coated stent-grafts was retrieved after 24 h (Example 3b and 3c devices were initially loaded with 25 and 150 µg respectively, see Examples 3b and 3c).

### Example 11: Paclitaxel-organic additive release profile post-sterilization - coated stent graft

The coated stents of Examples 3a, 3b and 3c were sterilized using ethylene oxide as set out in Test Method D. Following sterilization, the stent-grafts were immersed in an aqueous acetate buffer containing 30 % cyclodextrin. The release profile of paclitaxel from the device was measured over 24 hours at 37 °C according to Test Method M and the results are shown in Table 8.

**Table 8 - Paclitaxel content in media - post-sterilization**

| Prepared in example: | Paclitaxel content in media* [ug] | | | | | | | | |
|---|---|---|---|---|---|---|---|---|---|
| Timepoint [h] | 0.25 | 0.5 | 1 | 1.5 | 2 | 4 | 6 | 8 | 24 |
| Example 3b | 8 | 10 | 13 | 15 | 16 | 20 | 22 | 22 | 23 |
| Example 3c | 23 | 27 | 39 | 51 | 63 | 84 | 100 | 109 | 119 |

| | | | | | | | | | |
|---|---|---|---|---|---|---|---|---|---|
| Mean of N=2 * Content in media according to Test Methods M and C-II | | | | | | | | | |

Surprisingly, an average of 86 % of the PTX load of the coated stent-grafts was retrieved, showing that sterilization has no significant impact on the paclitaxel release profile (as compared with the release profile of the non-sterilized devices of Example 10).

### Example 12: Heparin activity of first coating layer - coated stent

Heparin activity of the underlying heparin bonded surface (first coating layer) of the compacted and deployed stents of Example 6 was measured according to WO2009/064372 (Test method J). The paclitaxel-caffeine coating (second particulate coating layer) was first extracted from the vascular stent surface by immersion in a glass vial containing 0.2% acetic acid in methanol, with shaking at 300rpm for 1hr at 40°C. The washed stents demonstrated therapeutically useful heparin activities of greater than 1 pmol/cm².

These results attest to the surprising activity of the heparin-bonded surface (first coating layer) under the conditions of coating with a paclitaxel-organic additive layer (second particulate coating layer), mechanical stress including compaction and expansion, and mechanical shear including deployment.

### Example 13: Heparin activity of first coating layer post sterilization - coated stent

Stents coated as in Example 2 are sterilized by ethylene oxide. The stents can subsequently undergo compaction and deployment to examine durability and robustness of the coating along with retention of heparin activity.

### Example 14: Dual activity test: Acute tissue transfer of the second particulate coating layer; and heparin activity of first coating layer - coated stent

Stent Device number 3 of Example 6 was examined for its ability to transfer paclitaxel from the stent surface to a vascular tissue in an *in vitro* model as described in Test Method A. The stent was removed, and vessel was analyzed for paclitaxel content using LC/MS-MS according to General Procedures. Approximately 16 µg paclitaxel per gram tissue was transferred from the stent surface to the porcine vascular tissue.

These paclitaxel tissue levels were within the reported therapeutic range of paclitaxel-coated vascular stents of 20 ug paclitaxel per gram tissue at 24 hrs, as described in the literature (M.D. Dake et al., J Vase Interven Rad, 22(5): 603-610, 2011, "Polymer-free Paclitaxel-coated Zilver PTX Stents-Evaluation of Pharmacokinetics and Comparative Safety in Porcine Arteries")

Heparin activity of the stent after it was removed from the vessel was measured according to Example 12, and was determined to be a therapeutically useful heparin activity of greater than 1 pmole/cm².

Thus, when a vascular stent with a coating of immobilized heparin (first coating layer) overcoated with a paclitaxel-organic additive coating (second particulate coating layer) was contacted with vascular tissue, a therapeutic amount of paclitaxel was transferred from the coating to the vascular tissue and a therapeutically useful heparin activity was retained for the heparin-bonded surface. Thus, an implantable medical device of the invention has the potential to exhibit dual therapeutic activity, when a first coating comprising heparin is applied to the stent, followed by an over coat of the paclitaxel-organic additive coating layer.

### Example 15: Effect of the second particulate coating layer on the heparin bioactivity of the first coating layer; pre- and post-sterilization - coated stent-graft

The heparin bioactivity of stent-grafts prepared as described in Example 3a, 3b and 3c was determined according to Test Method K and compared with the heparin bioactivity of a stent-graft with an immobilized heparin coating (first coating layer) but no paclitaxel-organic additive layer (no second particulate coating layer), referred to as the Heparin reference. The results are shown in Table 9.

**Table 9 - Relative heparin bioactivity of stent-grafts of the invention compared with heparin reference**

| **Prepared in example:** | **Relative heparin bioactivity* (%)** |
|---|---|
| Heparin reference | 100 |
| Example 3a | 78 |
| Example 3b | 86 |
| Example 3c | 92 |

| | |
|---|---|
| Mean of N=2 * Heparin Bioactivity according to Test Method K | |

It is evident from the results in Table 9 that a small decrease in heparin bioactivity (average 14.7%) was observed for stent-grafts coated with the second particulate coating layer (as compared to the heparin reference with only the first coating layer of immobilized heparin).

The heparin activity of the stent-grants was also determined post-sterilization using ethylene oxide according to Test Method D. The results are shown in Table 10.

**Table 10 - Heparin bioactivity before and after sterilization**

| **Prepared in example:** | **Relative heparin bioactivity* pre sterilization (%)** | **Relative heparin bioactivity* post sterilization** (%)** |
|---|---|---|
| Example 3a | 100 | 94 |
| Example 3b | 100 | 92 |
| Example 3c | 100 | 83 |
| EO reference*** | 100 | 30 |

| | | |
|---|---|---|
| Mean of N=2 * Heparin Bioactivity according to Test Method K ** Sterilization according to Test Method D *** Mean of N=4 | | |

Surprisingly, essentially no loss in heparin bioactivity was observed for stent-grafts that were subsequently subjected to sterilization according using ethylene oxide. The EO reference sample consisting of PVC tubing coated with immobilized heparin moiety lost 70% of its heparin bioactivity when subjected to Test Method D.

### Example 16: Heparin density of the first coating layer pre- and post-sterilization - coated stent graft

Stent-grafts prepared as described in Examples 3a, 3b and 3c were compared pre- and post-sterilization with ethylene oxide (according to Test Method D) with respect to their heparin density, using the procedure set out in Test Method L. The results are shown in Table 11.

**Table 11 - Heparin density pre-and post-sterilization**

| **Prepared in example:** | **Mean heparin density* pre sterilization** [µg/cm²]** | **Mean heparin density* post sterilization** [µg/cm²]** | **% Loss of heparin density post sterilization** |
|---|---|---|---|
| Example 3a | 8.9 ± 0.6 | 9.3 ± 0.8 | 0 |
| Example 3b | 10.1 ± 0.4 | 9.6 ± 0.5 | 5 |
| Example 3c | 9.4 ± 0.3 | 9.5 ± 0.4 | 0 |

| | | | |
|---|---|---|---|
| Values are mean of N=2 * Heparin Density according to Test Method L ** Sterilization according to Test Method D | | | |

Surprisingly, a comparatively small loss in heparin density was observed for stent-grafts that were subjected to sterilization.

### Example 17: Acute tissue transfer of second particulate coating layer - coated stent-graft

Stent-grafts prepared according to Examples 3a, 3b and 3c were examined for their ability to transfer paclitaxel from the stent-graft surface (specifically the second particular coating layer) to a vascular tissue in an *in vitro* model as described in Test Method A. Following removal of the stent-grafts from the vessel after 24 h, the vessels were analyzed for paclitaxel content using UPLC techniques (Test Method C-II).

**Table 12 - Paclitaxel (PTX) transfer from device to vascular tissue**

| **Prepared in example:** | **PTX content* on device as coated [µg] (Reference)** | **PTX content* on device pre test** [µg]** | **PTX content* on device post test** [µg]** | **PTX content* in tissue [µg/g]** |
|---|---|---|---|---|
| Example 3a | 471 ± 4.4 | 347 ± 16.8 | 176 ± 0.7 | 25 (N=1) |
| Example 3b | 23.8 ± 0.7 | 19.1 ± 3.2 | 10.6 ± 0.1 | 8.2 ± 2.9 |
| Example 3c | 155 ± 2.7 | 128 ± 0.8 | 89.3 ± 7.9 | 24 (N=1) |

| | | | | |
|---|---|---|---|---|
| Values are mean of N=2 * PTX content according to Test Method C-II ** PTX transfer from device to tissue according to Test Method A | | | | |

As can be seen from Table 12, approximately 8.2-25 µg paclitaxel per gram tissue was transferred from the stent-graft surface to the porcine vascular tissue. The test shows that paclitaxel can migrate from the implantable device into the vessel wall in therapeutically relevant levels.

### Example 18: Acute tissue transfer of second particulate coating layer - coated stent graft post-sterilization

Stent-grafts prepared according to Examples 3a, 3b and 3c were sterilized using Test Method D and then examined for their ability to transfer paclitaxel from the stent-graft surface according to Test Method A. As can be seen from Table 13, approximately 18 - 41 µg paclitaxel per gram tissue was transferred from the stent-graft surface to the porcine vascular tissue.

**Table 13 - Paclitaxel (PTX) transfer from device to vascular tissue, post-sterilization**

| **Prepared in example:** | **PTX content* on device as coated [µg] (Reference)** | **PTX content* on device pre test** [µg]** | **PTX content* on device post test** [µg]** | **PTX content* in tissue [µg/g]** |
|---|---|---|---|---|
| Example 3a | 473 ± 0.3 | 359 ± 32 | 172 ± 35 | 41 (N=1) |
| Example 3b | 24.8 ± 0.2 | 22.0 ± 1.6 | 10.0 ± 1.3 | 18 ± 0.1 |
| Example 3c | 150 ± 0.3 | 124 ± 10.6 | 64. 0 ± 2.9 | 35 ± 14 |

| | | | | |
|---|---|---|---|---|
| Values are mean of N=2 * PTX content according to Test Method C-II ** PTX transfer from device to tissue according to Test Method A | | | | |

The test shows that paclitaxel can migrate from the implantable device into the vessel wall in therapeutically relevant levels. Also, the migration of paclitaxel is not affected by the sterilization process according to Test Method D.

### Example 19: Heparin bioactivity of stent-grafts subjected to manipulation and In vitro tissue transfer and uptake, pre and post sterilization

The heparin bioactivity of stent-grafts prepared as described in Examples 3a, 3b and 3c was analysed according to Test Method K. Stent-grafts prepared as described in Examples 3a, 3b and 3c were manipulated according to Test Methods I-I and I-II and then subjected to Test Method A. Again, the heparin bioactivity was analysed according to Test Method K. The heparin bioactivity results of both experiments are shown in Table 14.

**Table 14 - Heparin bioactivity of stent-graft before and after: manipulation according to Test Methods I-I and I-II followed by Test Method A**

| **Prepared in example:** | **Relative Heparin bioactivity* pre-Test Methods I-I, I-II and Test Method A (%)** | **Relative heparin bioactivity* post Test Methods I-I, I-II and Test Method A (%)** |
|---|---|---|
| Example 3a | 100 | 60 |
| Example 3b | 100 | 66 |
| Example 3c | 100 | 63 |

| | | |
|---|---|---|
| Values are mean of N=2 * Heparin bioactivity according to Test Method K | | |

The experiment was repeated but the stent-grafts prepared as described in Examples 3a, 3b and 3c were sterilized using ethylene oxide according to Test Method D prior to being subjected to Test Methods I-I, I-II and Test Method A. The results are shown in Table 15.

**Table 15 - Heparin bioactivity of stent-graft before and after: sterilization according to Test Method D, then manipulation according to Test Methods I-I and I-II followed by Test Method A**

| **Prepared in example:** | **Relative Heparin bioactivity* pre-Test Methods D, I-I, I-II and Test Method A (%)** | **Relative heparin bioactivity* post Test Methods D, I-I, I-II and Test Method A (%)** |
|---|---|---|
| Example 3a | 100 | 73 |
| Example 3b | 100 | 64 |
| Example 3c | 100 | 55 |

| | | |
|---|---|---|
| Values are mean of N=2 * Heparin bioactivity according to Test Method K | | |

It is evident from the results of Tables 14 and 15 that the tested stent-grafts retained a high proportion of their initial heparin bioactivity following compaction, constraining and deployment (Test Methods I-I and I-II) and subsequent *in vitro* tissue transfer and uptake testing (Test Method A). Essentially no difference was observed between stent-grafts which were pre-sterilized (using Test Method D) and those that were not, indicating that the main loss of heparin bioactivity occurs as the device is subjected to Test Methods I-I and I-II, followed by Test Method A.

Thus, a coated stent-graft of the invention has the potential to exhibit dual therapeutic activity: transfer of a therapeutic amount of paclitaxel (from the second particulate coating layer) to the vascular tissue (as illustrated by Test Method A, and evidenced by Example 17) while a therapeutically useful amount of heparin bioactivity is retained on the immobilized heparin surface (first coating layer). Sterilization does not significantly affect the retained heparin bioactivity.

### Example 20: Blood contact activation

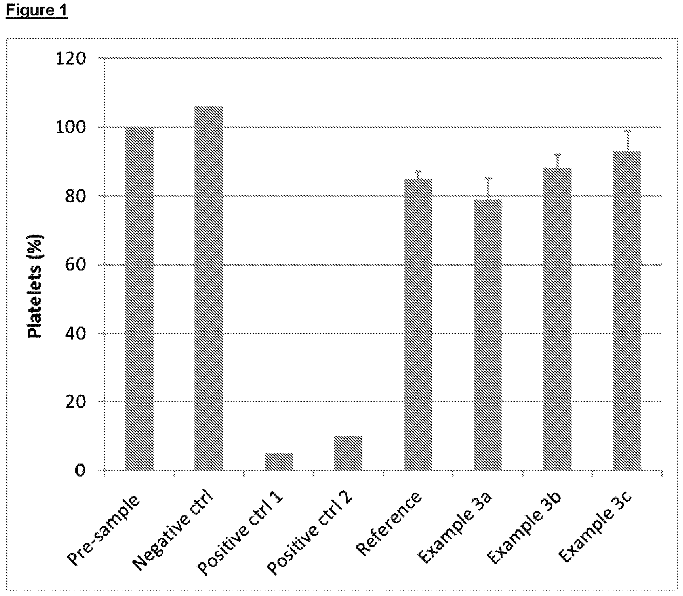

Stent-grafts prepared as described in Examples 3a, 3b and 3c were evaluated according to Test Method B. The platelet consumption of these stent-grafts of the invention was compared to platelet levels of a reference stent-graft (a stent-graft with only first coating layer and no paclitaxel-organic additive (second particulate coating layer)), and with a pre-sample (a heparinized PVC tubing) and the results are shown in Figure 1.

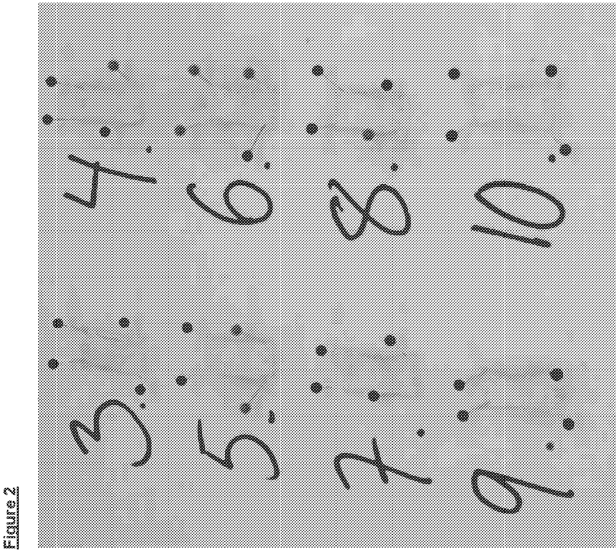

It was found that there were no clot formations in any the stent-grafts. All stent-grafts had similar platelet preservation, and there was no difference between the reference stent-graft and those coated according to Examples 3a, 3b and 3c. Also, visual examination of the luminal side of the stent-graft showed no clot formation, as shown in Figure 2. The numbering shown in Figure 2 correlates to the different coatings of the invention. Numbers 9 and 10 are coated according to Example 3a, numbers 5 and 6 are coated according to Example 3b, numbers 7 and 8 are coated according to Example 3c, and numbers 3 and 4 are reference devices coated with just the first coating layer of immobilized heparin moiety. Thus, there was no difference in thromboresistance between the reference stent-graft compared with those coated with according to Examples 3a, 3b and 3c, indicating that the application of the second particulate coating layer does not significantly impact the thromboresistance of the first coating layer.

### Example 21: SEM analysis of stent-graft pre- and post-manipulation

Scanning electron microscopy was performed according to techniques described under Evaluation Methods. Stent-graft prepared according to Example 3c was analyzed before and after manipulation according to Test Methods I-II and I-II. The abluminal side of the implantable device carrying the first and second coating layer) pre-manipulation is shown in Figure 3 and the abluminal side of the implantable device (carrying the first and second coating layer) post-manipulation is shown in Figure 4. The fact that no visual damage is evident post-manipulation indicates that the adhesion of the coating is surprisingly good, considering the amount of internal stress applied to the coating during the manipulation process.

### Example 22: Toluidine Blue staining of stent-grafts of the invention

Stent-grafts prepared as described in Example 3c were evaluated according to Test Method N, and compared with a reference stent-graft (stent-graft with a coating of immobilized heparin (first coating layer) but no second particulate coating layer). It was observed that the uniform coverage of the immobilized heparin layer (first coating layer) on the luminal side is not damaged by the addition of the second particulate coating layer on the abluminal side since the two stent-grafts stains equally darkly on the luminal side (dark areas being indicative of the immobilized heparin). The dark staining was seen throughout the whole luminal side of the device.

### Example 23: Application of dye-solvent formulation to a stent-graft with and without a first coating layer of immobilized heparin

Stent-grafts with a layer of immobilized heparin (first coating layer) were evaluated in terms of wettability and distribution by application of a dye-solvent formulation. Application of the formulation was performed essentially as described in Example 3c, but interchanging the second particulate coating layer with Patent Blue V dye. The results are shown in Figure 5 wherein the poor wettability of the solvent used for dissolving PTX-excipient and casting of the formulation onto the implantable device with no first layer (left stent-graft) is illustrated by areas of high concentration of dye formed on the device. The dye is more evenly distributed on the device that contains the first coating layer of immobilized heparin (right stent-graft). This example clearly shows that the first coating layer comprising immobilized heparin is capable of improving the even distribution of a subsequent coating to the device, which it is envisaged may be extrapolated to the distribution of the second particulate coating layer containing of paclitaxel. This should allow improved, even distribution of paclitaxel to surrounding tissue.

## Claims

1. An implantable medical device with a surface having a coating comprising:
a first coating layer comprising an immobilized heparin moiety and
a second particulate coating layer comprising elutable paclitaxel and at least one organic additive,
wherein at least a portion of the second particulate coating layer is in contact with at least a portion of the first coating layer.

2. An implantable medical device according to claim 1, wherein the medical device is a tubular medical device.

3. An implantable medical device according to claim 1 or claim 2, wherein the first coating layer comprises a polymer, wherein suitably the heparin moiety is covalently attached to the polymer, for example covalently end-point attached through its reducing end.

4. An implantable medical device according to claim 3, wherein the polymer is a cationic polymer.

5. An implantable medical device according to any one of claims 1 to 4, wherein the or each organic additive is non-polymeric and hydrolytically stable.

6. An implantable medical device according to any one of claims 1 to 5, wherein at least a proportion of the second particulate coating layer comprising paclitaxel and at least one organic additive melts as a single phase at a lower temperature than the melting point of paclitaxel and the at least one organic additive when in pure form;
and/or the second particulate coating layer is surfactant-free;
and/or the second particulate coating layer is polymer-free;
and/or the or each organic additive are in crystalline form.

7. An implantable medical device according to any one of claims 1 to 6, wherein the or each organic additive is independently selected from the list consisting of p-aminobenzoic acid, saccharin, ascorbic acid, methyl paraben, caffeine, calcium salicylate, pentetic acid, creatinine, ethylurea, acetaminophen, aspirin, theobromine, tryptophan, succinic acid, adipic acid, glutaric acid, theophylline, and saccharin sodium, and is suitably independently selected from the list consisting of p-aminobenzoic acid, methyl paraben, caffeine, calcium salicylate and succinic acid, for example is caffeine or succinic acid.

8. An implantable medical device according to any one of claims 1 to 7, wherein the implantable medical device is a stent and is suitably composed of a metal alloy such as nitinol or stainless steel;
or the implantable medical device is a stent-graft, wherein the stent-graft comprises a stent member and a graft member.

9. An implantable medical device according to any one of claims 1 to 8, wherein the coating is applied to a surface of the device which is composed of ePTFE.

10. An implantable medical device according to any one of claims 1 to 9, wherein at least a portion of the second particulate coating layer is applied on top of at least a portion of the first coating layer.

11. An implantable medical device according to any one of claims 1 to 10, wherein the first coating layer is applied to the medical device before the second particulate coating layer.

12. An implantable medical device according to any one of claims 1 to 11, wherein the second particulate coating layer is applied to the medical device by dissolving the paclitaxel and the at least one organic additive in a solvent to form a solution, applying the solution to the medical device and then evaporating the solvent, wherein the solvent is suitably selected from water, acetone and mixtures thereof.

13. A process for preparing a coated implantable medical device comprising the steps of:
i) treating the medical device to provide a first coating layer comprising an immobilized heparin moiety; and further
ii) treating the medical device to provide a second particulate coating layer comprising elutable paclitaxel and at least one organic additive,
wherein at least a portion of the second particulate coating layer is in contact with at least a portion of the first coating layer.

14. A process according to claim 13, wherein step i) comprises the steps of:
a) treating the medical device to provide a polymer coating layer; and then
b) reacting said polymer layer with a heparin moiety to immobilize the heparin moiety to the polymer coating layer.

15. A process according to claim 13 or claim 14, wherein in step ii) the second particulate coating layer is applied to the medical device by dissolving the paclitaxel and the at least one organic additive in a solvent to form a solution, applying the solution to the medical device and then evaporating the solvent, wherein the solvent is suitably selected from water, acetone and mixtures thereof.

16. A process according to claim 14, wherein the heparin moiety is covalently attached to the polymer coating layer, suitably covalently end-point attached to the polymer coating layer wherein the end point attached heparin moiety is connected through its reducing end.

## Patentansprüche

1. Implantierbare medizinische Vorrichtung mit einer Oberfläche mit einem Überzug, umfassend:
eine erste Überzugsschicht, die eine immobilisierte Heparineinheit umfasst, und
eine zweite partikuläre Überzugsschicht, die eluierbares Paclitaxel und mindestens einen organischen Zusatzstoff umfasst,
wobei mindestens ein Teil der zweiten partikulären Überzugsschicht mit mindestens einem Teil der ersten Überzugsschicht in Kontakt ist.

2. Implantierbare medizinische Vorrichtung nach Anspruch 1, wobei die medizinische Vorrichtung eine röhrenförmige medizinische Vorrichtung ist.

3. Implantierbare medizinische Vorrichtung nach Anspruch 1 oder Anspruch 2, wobei die erste Überzugsschicht ein Polymer umfasst, wobei die Heparineinheit geeigneterweise kovalent an das Polymer gebunden ist, beispielsweise kovalent über sein reduzierendes Ende terminal gebunden ist.

4. Implantierbare medizinische Vorrichtung nach Anspruch 3, wobei das Polymer ein kationisches Polymer ist.

5. Implantierbare medizinische Vorrichtung nach einem der Ansprüche 1 bis 4, wobei der oder jeder organische Zusatzstoff nicht polymer und hydrolytisch stabil ist.

6. Implantierbare medizinische Vorrichtung nach einem der Ansprüche 1 bis 5, wobei mindestens ein Teil der zweiten partikulären Überzugsschicht, die Paclitaxel und mindestens einen organischen Zusatzstoff umfasst, als eine einzige Phase bei einer niedrigeren Temperatur als der Schmelzpunkt von Paclitaxel und dem mindestens einen organischen Zusatzstoff schmilzt, wenn sie in reiner Form vorliegen;
und/oder die zweite partikuläre Überzugsschicht tensidfrei ist;
und/oder die zweite partikuläre Überzugsschicht polymerfrei ist;
und/oder der oder jeder organische Zusatzstoff in kristalliner Form vorliegt.

7. Implantierbare medizinische Vorrichtung nach einem der Ansprüche 1 bis 6, wobei der oder jeder organische Zusatzstoff unabhängig aus der Liste ausgewählt ist, die aus p-Aminobenzoesäure, Saccharin, Ascorbinsäure, Methylparaben, Koffein, Calciumsalicylat, Pentetsäure, Kreatinin, Ethylharnstoff, Acetaminophen, Aspirin, Theobromin, Tryptophan, Bernsteinsäure, Adipinsäure, Glutarsäure, Theophyllin und Saccharin-Natrium besteht, und geeigneterweise unabhängig aus der Liste ausgewählt ist, die aus p-Aminobenzoesäure, Methylparaben, Koffein, Calciumsalicylat und Bernsteinsäure besteht, beispielsweise Koffein oder Bernsteinsäure ist.

8. Implantierbare medizinische Vorrichtung nach einem der Ansprüche 1 bis 7, wobei die implantierbare medizinische Vorrichtung ein Stent ist und geeigneterweise aus einer Metalllegierung wie etwa Nitinol oder rostfreiem Stahl besteht;
oder die implantierbare medizinische Vorrichtung ein Stentgraft ist, wobei der Stentgraft ein Stentelement und ein Graftelement umfasst.

9. Implantierbare medizinische Vorrichtung nach einem der Ansprüche 1 bis 8, wobei der Überzug auf eine Oberfläche der Vorrichtung aufgebracht ist, die aus ePTFE besteht.

10. Implantierbare medizinische Vorrichtung nach einem der Ansprüche 1 bis 9, wobei mindestens ein Teil der zweiten partikulären Überzugsschicht oben auf mindestens einen Teil der ersten Überzugsschicht aufgebracht ist.

11. Implantierbare medizinische Vorrichtung nach einem der Ansprüche 1 bis 10, wobei die erste Überzugsschicht vor der zweiten partikulären Überzugsschicht auf die medizinische Vorrichtung aufgebracht wird.

12. Implantierbare medizinische Vorrichtung nach einem der Ansprüche 1 bis 11, wobei die zweite partikuläre Überzugsschicht auf die medizinische Vorrichtung aufgebracht wird, indem das Paclitaxel und der mindestens eine organische Zusatzstoff in einem Lösungsmittel aufgelöst werden, um eine Lösung zu bilden, die Lösung auf die medizinische Vorrichtung aufgebracht wird und dann das Lösungsmittel verdampft wird, wobei das Lösungsmittel geeigneterweise aus Wasser, Aceton und Mischungen davon ausgewählt ist.

13. Verfahren zum Herstellen einer beschichteten implantierbaren medizinischen Vorrichtung, umfassend die Schritte:
i) Behandeln der medizinischen Vorrichtung, um eine erste Überzugsschicht bereitzustellen, die eine immobilisierte Heparineinheit umfasst; und ferner
ii) Behandeln der medizinischen Vorrichtung, um eine zweite partikuläre Überzugsschicht bereitzustellen, die eluierbares Paclitaxel und mindestens einen organischen Zusatzstoff umfasst,
wobei mindestens ein Teil der zweiten partikulären Überzugsschicht mit mindestens einem Teil der ersten Überzugsschicht in Kontakt ist.

14. Verfahren nach Anspruch 13, wobei Schritt i) die folgenden Schritte umfasst:
a) Behandeln der medizinischen Vorrichtung, um eine Polymerüberzugsschicht bereitzustellen; und dann
b) Umsetzen der Polymerschicht mit einer Heparineinheit, um die Heparineinheit an der Polymerüberzugsschicht zu immobilisieren.

15. Verfahren nach Anspruch 13 oder Anspruch 14, wobei in Schritt ii) die zweite partikuläre Überzugsschicht auf die medizinische Vorrichtung aufgebracht wird, indem das Paclitaxel und der mindestens eine organische Zusatzstoff in einem Lösungsmittel aufgelöst werden, um eine Lösung zu bilden, die Lösung auf die medizinische Vorrichtung aufgebracht wird und dann das Lösungsmittel verdampft wird, wobei das Lösungsmittel geeigneterweise aus Wasser, Aceton und Mischungen davon ausgewählt ist.

16. Verfahren nach Anspruch 14, wobei die Heparineinheit kovalent an die Polymerüberzugsschicht gebunden ist, geeigneterweise kovalent terminal an die Polymerüberzugsschicht gebunden ist, wobei die terminal gebundene Heparineinheit über ihr reduzierendes Ende verbunden ist.

## Revendications

1. Dispositif médical implantable avec une surface ayant un revêtement comprenant :
une première couche de revêtement comprenant un fragment héparine immobilisé et
une seconde couche de revêtement particulaire comprenant du paclitaxel éluable et au moins un additif organique,
dans lequel au moins une partie de la seconde couche de revêtement particulaire est en contact avec au moins une partie de la première couche de revêtement.

2. Dispositif médical implantable selon la revendication 1, dans lequel le dispositif médical est un dispositif médical tubulaire.

3. Dispositif médical implantable selon la revendication 1 ou la revendication 2, dans lequel la première couche de revêtement comprend un polymère, dans lequel de manière appropriée le fragment héparine est attaché de manière covalente au polymère, par exemple attaché par point d'extrémité de manière covalente à travers son extrémité réductrice.

4. Dispositif médical implantable selon la revendication 3, dans lequel le polymère est un polymère cationique.

5. Dispositif médical implantable selon l'une quelconque des revendications 1 à 4, dans lequel le ou chaque additif organique est non polymère et stable hydrolytiquement.

6. Dispositif médical implantable selon l'une quelconque des revendications 1 à 5, dans lequel au moins une proportion de la seconde couche de revêtement particulaire comprenant du paclitaxel et au moins un additif organique fond sous la forme d'une seule phase à une température inférieure au point de fusion de paclitaxel et de l'au moins un additif organique lorsqu'il est sous forme pure ;
et/ou la seconde couche de revêtement particulaire est exempte de tensioactif ;
et/ou la seconde couche de revêtement particulaire est exempte de polymère ;
et/ou le ou chaque additif organique est sous forme cristalline.

7. Dispositif médical implantable selon l'une quelconque des revendications 1 à 6, dans lequel le ou chaque additif organique est choisi indépendamment dans la liste constituée de l'acide p-aminobenzoïque, de la saccharine, de l'acide ascorbique, du méthylparabène, de la caféine, du salicylate de calcium, de l'acide pentétique, de la créatinine, de l'éthylurée, de l'acétaminophène, de l'aspirine, de la théobromine, du tryptophane, de l'acide succinique, de l'acide adipique, de l'acide glutarique, de la théophylline et de la saccharine sodique, et est convenablement choisi indépendamment dans la liste constituée de l'acide p-aminobenzoïque, du méthylparabène, de la caféine, du salicylate de calcium et de l'acide succinique, par exemple est la caféine ou l'acide succinique.

8. Dispositif médical implantable selon l'une quelconque des revendications 1 à 7, dans lequel le dispositif médical implantable est un stent et est composé de manière appropriée d'un alliage métallique tel que du nitinol ou de l'acier inoxydable ;
ou le dispositif médical implantable est un stent-greffe, dans lequel le stent-greffe comprend un élément stent et un élément greffon.

9. Dispositif médical implantable selon l'une quelconque des revendications 1 à 8, dans lequel le revêtement est appliqué sur une surface du dispositif qui est composée d'ePTFE.

10. Dispositif médical implantable selon l'une quelconque des revendications 1 à 9, dans lequel au moins une partie de la seconde couche de revêtement particulaire est appliquée sur au moins une partie de la première couche de revêtement.

11. Dispositif médical implantable selon l'une quelconque des revendications 1 à 10, dans lequel la première couche de revêtement est appliquée sur le dispositif médical avant la seconde couche de revêtement particulaire.

12. Dispositif médical implantable selon l'une quelconque des revendications 1 à 11, dans lequel la seconde couche de revêtement particulaire est appliquée au dispositif médical en dissolvant le paclitaxel et l'au moins un additif organique dans un solvant pour former une solution, en appliquant la solution au dispositif médical puis en évaporant le solvant, dans lequel le solvant est choisi de manière appropriée parmi l'eau, l'acétone et leurs mélanges.

13. Procédé de préparation d'un dispositif médical implantable revêtu comprenant les étapes suivantes :
i) traitement du dispositif médical pour fournir une première couche de revêtement comprenant un fragment héparine immobilisé ; et autre
ii) traitement du dispositif médical pour fournir une seconde couche de revêtement particulaire comprenant du paclitaxel éluable et au moins un additif organique,
dans lequel au moins une partie de la seconde couche de revêtement particulaire est en contact avec au moins une partie de la première couche de revêtement.

14. Procédé selon la revendication 13, dans lequel l'étape i) comprend les étapes suivantes :
a) traitement du dispositif médical pour fournir une couche de revêtement polymère ; et puis
b) réaction de ladite couche polymère avec un fragment héparine pour immobiliser le fragment héparine sur la couche de revêtement polymère.

15. Procédé selon la revendication 13 ou la revendication 14, dans lequel à l'étape ii) la seconde couche de revêtement particulaire est appliquée au dispositif médical en dissolvant le paclitaxel et l'au moins un additif organique dans un solvant pour former une solution, en appliquant la solution au dispositif médical puis en évaporant le solvant, dans lequel le solvant est choisi de manière appropriée parmi l'eau, l'acétone et leurs mélanges.

16. Procédé selon la revendication 14, dans lequel le fragment héparine est attaché de manière covalente à la couche de revêtement polymère, attaché par point d'extrémité de manière covalente et de manière appropriée à la couche de revêtement polymère dans lequel le fragment héparine attaché par point d'extrémité est connecté à travers son extrémité réductrice.
